(19) Europäisches Patentamt
European Patent Office
Office européen des brevets

(11) EP 1 312 344 B1

(12) FASCICULE DE BREVET EUROPEEN

(45) Date de publication et mention de la délivrance du brevet:
**12.01.2011 Bulletin 2011/02**

(51) Int Cl.:
***A61K 8/898*** *(2006.01)* ***A61Q 5/10*** *(2006.01)*

(21) Numéro de dépôt: **02292743.8**

(22) Date de dépôt: **04.11.2002**

(54) **Composition de teinture pour fibres kératiniques comprenant une silicone aminée particulière**

Zusammensetzung zur Färbung von Keratinfaser, die spezielle Aminosilikonen enthält

Dyeing composition for keratinous fibres containing aminosilicone

(84) Etats contractants désignés:
**AT BE BG CH CY CZ DE DK EE ES FI FR GB GR IE IT LI LU MC NL PT SE SK TR**

(30) Priorité: **08.11.2001 FR 0114467**

(43) Date de publication de la demande:
**21.05.2003 Bulletin 2003/21**

(73) Titulaire: **L'Oréal**
**75008 Paris (FR)**

(72) Inventeurs:
- **LeGrand, Frédéric**
  **92400 Courbevoie (FR)**
- **Milleqant, Jean-Marie**
  **94100 Saint-Maur-des Fosses (FR)**

(74) Mandataire: **Fevrier, Murielle Françoise E.**
**L'Oréal**
**D.I.P.I.**
**25-29 Quai Aulagnier**
**92600 Asnières-sur-Seine Cedex (FR)**

(56) Documents cités:
**EP-A- 0 890 355** **DE-A- 19 754 053**
**GB-A- 2 165 550**

Remarques:
Le dossier contient des informations techniques présentées postérieurement au dépôt de la demande et ne figurant pas dans le présent fascicule.

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 1 312 344 B1

## Description

La présente invention concerne une composition de teinture des fibres kératiniques humaines et plus particulièrement des cheveux, comprenant au moins un colorant direct ou d'oxydation et au moins une silicone aminée particulière.

Il existe principalement deux types de coloration des fibres kératiniques. La coloration directe, mettant en oeuvre en présence ou en l'absence d'agents oxydants, des colorants directs et / ou des pigments qui sont des molécules colorées, conférant aux fibres une couleur temporaire qui s'estompe après quelques shampooings, et la coloration dite "coloration d'oxydation" mettant en oeuvre des précurseurs de colorants d'oxydation et un agent oxydant, qui confère aux fibres une couleur plus tenace que la précédente. L'utilisation d'un agent oxydant entraîne en général une certaine dégradation de la fibre kératinique. Le document EP11138317 décrit une composition de teinture pour les cheveux comprenant une silicone aminée correspondant à la formule (I) ci-dessous où $R_2$ représente OH, ou bien à la formule (II); les groupements terminaux $R_2$ des formules (5) et (6) sont choisis parmi le radical hydroxy (préféré), méthyl ou méthoxy.

On constate actuellement une tendance très nette à l'augmentation de la fréquence des shampooings, ce qui se traduit par une dégradation plus importante des colorations entre deux applications.

Il existe donc un besoin d'améliorer la ténacité des colorations directes ou d'oxydation, en particulier vis à vis des shampooings.

Après d'importantes études sur la question, la demanderesse a découvert de manière tout à fait inattendue et surprenante que l'utilisation d'une composition comprenant au moins un colorant direct ou au moins un colorant d'oxydation et en outre une silicone aminée particulière, permettait de résoudre ce problème.

En outre, elle permet d'améliorer l'état de la fibre.
Par amélioration de l'état de la fibre, on entend une diminution de la porosité ou de la solubilité alcaline de la fibre et une amélioration des propriétés cosmétiques et en particulier du lissage, de la douceur et de la facilité de démêlage et de coiffage.
Cet effet est rémanent, c'est à dire durable.
La porosité se mesure par la fixation à 37°C et à pH10, en 2 minutes, de la 2-nitro paraphénylènediamine à 0,25% dans un mélange éthanol / tampon pH 10 (rapport volumique 10/90).

La solubilité alcaline correspond à la perte de masse d'un échantillon de 100 mg de fibres kératiniques sous l'action de la soude décinormale pendant 30 minutes à 65°C.

Cette découverte est à la base de la présente invention.

La présente invention a ainsi pour objet une composition de teinture pour fibres kératiniques humaines, et plus particulièrement des cheveux, comprenant, dans un milieu cosmétiquement acceptable, au moins un colorant direct ou au moins un colorant d'oxydation, qui est caractérisée par le fait qu'elle contient en outre au moins une silicone aminée de formules (I) ou (II) décrites ci-après.

Un autre objet de l'invention porte sur une composition prête à l'emploi pour la teinture des fibres kératiniques humaines qui contient au moins un colorant direct ou au moins un colorant d'oxydation et au moins une silicone aminée de formules (I) ou (II) décrites ci-après et un agent oxydant.
Par "composition prête à l'emploi", on entend, au sens de l'invention, la composition destinée à être appliquée telle quelle sur les fibres kératiniques, c'est à dire qu'elle peut être stockée telle quelle avant utilisation ou résulter du mélange extemporané de deux ou plusieurs compositions.

L'invention vise également un procédé de teinture des fibres kératiniques humaines et plus particulièrement des cheveux, consistant à appliquer sur les fibres au moins une composition colorante comprenant, dans un milieu cosmétiquement acceptable, au moins un colorant direct et en outre au moins une silicone aminée de formules (I) ou (II).

L'invention vise aussi un procédé de teinture des fibres kératiniques humaines et plus particulièrement des cheveux, consistant à appliquer sur les fibres au moins une composition colorante comprenant, dans un milieu cosmétiquement acceptable, au moins un colorant direct ou au moins un colorant d'oxydation, et en outre au moins une silicone aminée de formules (I) ou (II), la couleur étant révélée à pH alcalin, neutre ou acide, à l'aide d'une composition comprenant au moins un agent oxydant qui est mélangée juste au moment de l'emploi à la composition colorante ou qui est appliquée séquentiellement sans rinçage intermédiaire.

L'invention a également pour objet des dispositifs de teinture ou « kits » à plusieurs compartiments.

Un dispositif à 2 compartiments selon l'invention comprend un compartiment renfermant une composition comprenant, dans un milieu cosmétiquement acceptable, au moins un colorant direct ou au moins un colorant d'oxydation, et au moins une silicone aminée de formules (I) ou (II), et un autre compartiment renfermant une composition comprenant, dans un milieu cosmétiquement acceptable, un agent oxydant.

Un autre dispositif, à 3 compartiments selon l'invention, comprend un premier compartiment renfermant une composition comprenant, dans un milieu cosmétiquement acceptable, au moins un colorant direct ou au moins un colorant d'oxydation, un deuxième compartiment renfermant une composition comprenant, dans un milieu cosmétiquement acceptable, au moins un agent oxydant, et un troisième compartiment renfermant une composition comprenant, dans un milieu cosmétiquement acceptable, au moins une silicone aminée de formules (I) ou (II) décrites ci-après.

Mais d'autres caractéristiques, aspects, objets et avantages de l'invention apparaîtront encore plus clairement à la lecture de la description et des exemples qui suivent.

Silicones aminées

Les silicones aminées de formules (I) ou (II) selon l'invention sont les suivantes :

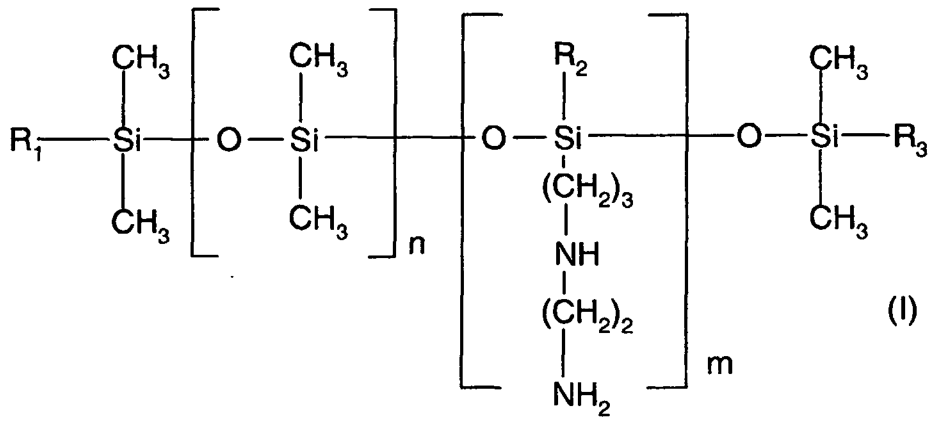

(I)

dans laquelle :

- m et n sont des nombres tels que la somme (n + m) peut varier notamment de 1 à 1 000 et en particulier de 50 à 250 et plus particulièrement de 100 à 200,
- n pouvant désigner un nombre de 0 à 999 et notamment de 49 à 249 et plus particulièrement de 125 à 175 et m pouvant désigner un nombre de 1 à 1 000, et notamment de 1 à 10 et plus particulièrement de 1 à 5;
- $R_1$, $R_2$, $R_3$, identiques ou différents, représentent un radical hydroxy ou alcoxy en $C_1$-$C_4$, l'un au moins des radicaux $R_1$ à $R_3$ désignant un radical alcoxy.

De préférence le radical alcoxy est un radical méthoxy.

Le rapport molaire Hydroxy/Alcoxy est de préférence compris entre 0,2:1 et 0,4:1 et de préférence entre 0,25:1 et 0,35: 1 et plus particulièrement est égal à 0,3.

La silicone aminée de formule (I) présente une masse moléculaire moyenne en poids allant de préférence de 2000 à 1000000 et encore plus particulièrement de 3500 à 200000.

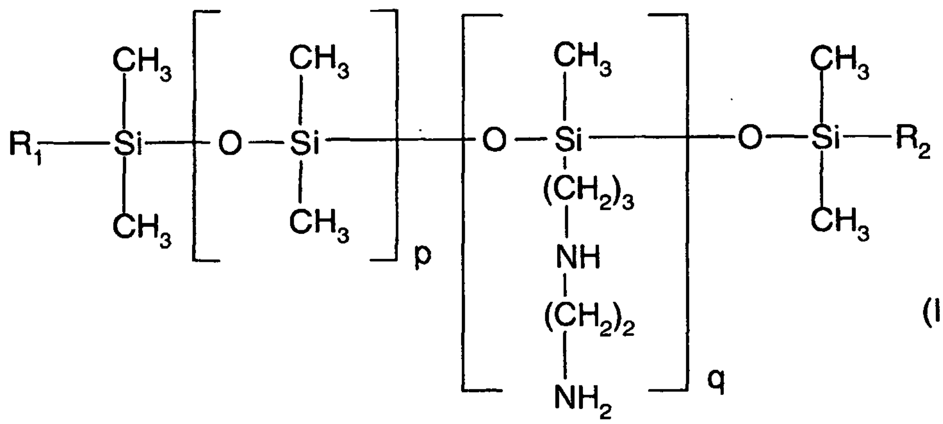

(II)

dans laquelle :

p et q sont des nombres tels que la somme (p + q) peut varier notamment de 1 à 1 000 et en particulier de 50 à 350, et plus particulièrement de 150 à 250

p pouvant désigner un nombre de 0 à 999 et notamment de 49 à 349 et plus particulièrement de 159 à 239 et q pouvant désigner un nombre de 1 à 1 000, et notamment de 1 à 10 et plus particulièrement de 1 à 5;

$R_1$, $R_2$, différents, représentent un radical hydroxy ou alcoxy en $C_1$-$C_4$, l'un au moins des radicaux $R_1$ à $R_2$ désignant un radical alcoxy.

De préférence le radical alcoxy est un radical méthoxy.

Le rapport molaire Hydroxy/Alcoxy est de préférence compris entre 1:0,8 et 1:1,1 et de préférence entre 1:0,9 et 1:1 et plus particulièrement est égal à 1:0,95.

La silicone aminée de formule (II) présente une masse moléculaire moyenne en poids allant de préférence de 2000 à 200.000, et encore plus particulièrement de 5.000 à 100.000 et plus particulièrement encore de 10.000 à 50.000.

Les masses moléculaires moyennes en poids de ces silicones aminées sont mesurées par Chromatographie par Perméation de Gel (GPC) à température ambiante en équivalent polystyrène. Les colonnes utilisées sont des colonnes μ styragel. L'éluant est le THF, le débit est de 1 ml/mn. On injecte 200 μl d'une solution à 0,5% en poids de silicone dans le THF. La détection se fait par réfractométrie et UVmétrie.

Les produits commerciaux correspondant à ces silicones de structure (I) ou (II) peuvent inclure dans leur composition une ou plusieurs autres silicones aminées dont la structure est différente des structures (I) et (II).

Un produit contenant des silicones aminées de structure (I) est proposé par la société WACKER sous la dénomination BELSIL ADM 652®.

Des produits contenant des silicones aminées de structure (II) sont proposés par la société WACKER sous la dénomination Fluid WR 1300®, et BELSIL ADM 6057®.

Lorsque ces silicones aminées sont mises en oeuvre, une forme de réalisation particulièrement intéressante est leur utilisation sous forme d'émulsion huile-dans-eau. L'émulsion huile-dans-eau peut comprendre un ou plusieurs tensioactifs. Les tensioactifs peuvent être de toute nature mais de préférence cationique et/ou non ionique.

Les particules de silicone dans l'émulsion ont une taille moyenne allant généralement de 3 nm à 500 nanomètres. De préférence, notamment pour les silicones aminées de formule (II), on utilise des microémulsions de taille allant de 5 nm à 60 nanomètres et plus particulièrement de 10 nm à 50 nanomètres.

On peut utiliser selon l'invention les microémulsions de silicones aminées de formule (II) proposées sous la dénomination FINISH CT 96 E® ou SLM 28020® par la société WACKER.

De préférence la silicone aminée de formule (I) ou (II) est choisie de telle façon que l'angle de contact avec l'eau d'un cheveu traité avec une composition contenant 2% MA (matières actives) de ladite silicone selon l'invention soit compris entre 90 et 180° et de préférence entre 90 et 130°, bornes incluses.

De préférence la composition contenant la ou les silicones aminées de formule (I) ou (II) est telle que l'angle de contact d'un cheveu traité avec la dite composition est compris entre 90 et 180° et de préférence entre 90 et 130°, bornes incluses.

La mesure de l'angle de contact est basée sur l'immersion d'un cheveu dans de l'eau distillée. Il consiste à évaluer la force exercée par l'eau sur le cheveu lors de son immersion dans l'eau distillée et lors de son retrait. Les forces ainsi mesurées sont directement reliées à l'angle de contact θ entre l'eau et la surface du cheveu. Le cheveu est dit hydrophile lorsque l'angle θ est compris entre 0 et 90°, hydrophobe lorsque cet angle est compris entre 90° et 180°, bornes incluses.

Le test s'effectue avec des mèches de cheveux naturels ayant été décolorés dans les mêmes conditions puis lavés.

Chaque mèche de 1 g est placée dans un cristallisoir de 75 mm de diamètre puis recouverte de façon homogène par 5 mL de la formule à tester. La mèche est ainsi laissée 15 minutes à température ambiante puis rincée pendant 30 secondes. La mèche essorée est laissée à l'air libre jusqu'à ce qu'elle soit complètement sèche.

Pour chaque évaluation, 10 cheveux ayant subi le même traitement sont analysés. Chaque échantillon, fixé à une microbalance de précision, est immergé par la pointe dans un récipient rempli d'eau distillée. Cette balance DCA (« Dynamic Contact Angle Analyser »), de la société CAHN Instruments, permet de mesurer la force (F) exercée par l'eau sur le cheveu.

Parallèlement, le périmètre du cheveu (P) est mesuré *via* une observation microscopique. La force moyenne de mouillabilité sur 10 cheveux et la section des cheveux analysés permettent d'obtenir l'angle de contact du cheveu sur l'eau, selon la formule :

$$F = P * \Gamma lv * \cos\theta$$

où F est la force de mouillabilité exprimée en Newton, P le périmètre du cheveu en mètre, Γlv la tension interfaciale liquide / vapeur de l'eau en J/m$^2$ et θ l'angle de contact.
Le produit SLM 28020® de WACKER à 12% dans l'eau (soit 2% en silicone aminée) conduit à un angle de contact de 93° selon le test indiqué ci-dessus.

La silicone aminée de formule (I) ou (II) est utilisée de préférence dans la composition de teinture selon l'invention en une quantité allant de 0,01 à 20% en poids du poids total de la composition. Plus préférentiellement, cette quantité va de 0,1 à 15% en poids et encore plus particulièrement de 0,5 à 10 % en poids.

<u>Colorants d'oxydation</u>

Les colorants d'oxydation utilisables selon l'invention sont choisis parmi les bases d'oxydation et/ou les coupleurs.
De préférence les compositions selon l'invention contiennent au moins une base d'oxydation.
Les bases d'oxydation utilisables dans le cadre de la présente invention sont choisies parmi celles classiquement connues en teinture d'oxydation, et parmi lesquelles on peut notamment citer les ortho- et para-phénylènediamines, les bases doubles, les ortho- et para- aminophénols, les bases hétérocycliques suivantes ainsi que leurs sels d'addition avec un acide.
On peut notamment citer :

- **(A)** les paraphénylènediamines de formule (III) suivante et leurs sels d'addition avec un acide :

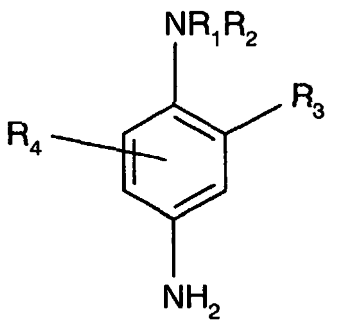

**(III)**

dans laquelle :

R$_1$ représente un atome d'hydrogène, un radical alkyle en C$_1$-C$_4$, monohydroxyalkyle en C$_1$-C$_4$, polyhydroxyalkyle en C$_2$-C$_4$, alcoxy(C$_1$-C$_4$)alkyle(C$_1$-C$_4$), alkyle en C$_1$-C$_4$ substitué par un groupement azoté, phényle ou 4'-aminophényle ;
R$_2$ représente un atome d'hydrogène, un radical alkyle en C$_1$-C$_4$, monohydroxyalkyle en C$_1$-C$_4$ ou polyhydroxyalkyle en C$_2$-C$_4$, alcoxy(C$_1$-C$_4$)alkyle(C$_1$-C$_4$) ou alkyle en C$_1$-C$_4$ substitué par un groupement azoté ;
R$_1$ et R$_2$ peuvent également former avec l'atome d'azote qui les porte un hétérocycle azoté à 5 ou 6 chaînons éventuellement substitué par un ou plusieurs groupements alkyle, hydroxy ou uréido;
R$_3$ représente un atome d'hydrogène, un atome d'halogène tel qu'un atome de chlore, un radical alkyle en C$_1$-C$_4$, sulfo, carboxy, monohydroxyalkyle en C$_1$-C$_4$ ou hydroxyalcoxy en C$_1$-C$_4$, acétylaminoalcoxy en C$_1$-C$_4$, mésylaminoalcoxy en C$_1$-C$_4$ ou carbamoylaminoalcoxy en C$_1$-C$_4$,
R$_4$ représente un atome d'hydrogène, d'halogène ou un radical alkyle en C$_1$-C$_4$.

Parmi les groupements azotés de la formule (III) ci-dessus, on peut citer notamment les radicaux amino, monoalkyl (C$_1$-C$_4$)amino, dialkyl(C$_1$-C$_4$)amino, trialkyl(C$_1$-C$_4$)amino, monohydroxyalkyl(C$_1$-C$_4$)amino, imidazolinium et ammonium.
Parmi les paraphénylènediamines de formule (III) ci-dessus, on peut plus particulièrement citer la paraphénylènediamine, la paratoluylènediamine, la 2-chloro-paraphénylènediamine, la 2,3-diméthyl-paraphénylènediamine, la 2,6-diméthyl-paraphénylènediamine, la 2,6-diéthyl-paraphénylènediamine, la 2,5-diméthyl-paraphénylènediamine, la N,N-diméthyl-paraphénylènediamine, la N,N-diéthyl-paraphénylènediamine, la N,N-dipropyl-paraphénylènediamine, la 4-amino-N,N-diéthyl-3-méthyl-aniline, la N,N-bis-(β-hydroxyéthyl)-paraphénylènediamine, la 4-N,N-bis-(β-hydroxyéthyl)amino-2-méthyl-aniline, la 4-N,N-bis-(β-hydroxyéthyl)-amino 2-chloro-aniline, la 2-β-hydroxyéthyl-paraphénylènediamine, la 2-fluoro-paraphénylènediamine, la 2-isopropyl-paraphénylènediamine, la N-(β-hydroxypropyl)-paraphénylènediamine, la 2-hydroxyméthyl-paraphénylènediamine, la N,N-diméthyl-3-méthylparaphénylènediamine, la N,N-(éthyl,β-hydroxyéthyl)-paraphénylènediamine, la N-(β,γ-dihydroxypropyl)-paraphénylènediamine, la N-(4'-aminophényl)-paraphénylènediamine, la N-phényl-paraphénylènediamine, la 2-β-hydroxyéthyloxy-paraphénylènediamine, la 2-β-acétylaminoéthyloxy-paraphénylènediamine, la N-(β-méthoxyéthyl)-paraphénylènediamine, 2-méthyl-1-N-β-hydroxyéthyl-paraphénylènediamine, et leurs sels d'addition avec un acide.
Parmi les paraphénylènediamines de formule (III) ci-dessus, on préfère tout particulièrement la paraphénylènediamine, la paratoluylènediamine, la 2-isopropyl-paraphénylènediamine, la 2-β-hydroxyéthyl-paraphénylènediamine, la 2-β-hydroxyéthyloxy-paraphénylènediamine, la 2,6-diméthyl-paraphénylène-diamine, la 2,6-diéthyl-paraphénylènediamine, la 2,3-diméthyl-paraphénylènediamine, la N,N-bis-(β-hydroxyéthyl)-paraphénylènediamine, la 2-chloro-paraphénylènediamine, et leurs sels d'addition avec un acide.

- **(B)** Selon l'invention, on entend par bases doubles, les composés comportant au moins deux noyaux aromatiques sur lesquels sont portés des groupements amino et/ou hydroxyle.
  Parmi les bases doubles utilisables à titre de bases d'oxydation dans les compositions tinctoriales conformes à l'invention, on peut notamment citer les composés répondant à la formule (IV) suivante, et leurs sels d'addition avec un acide :

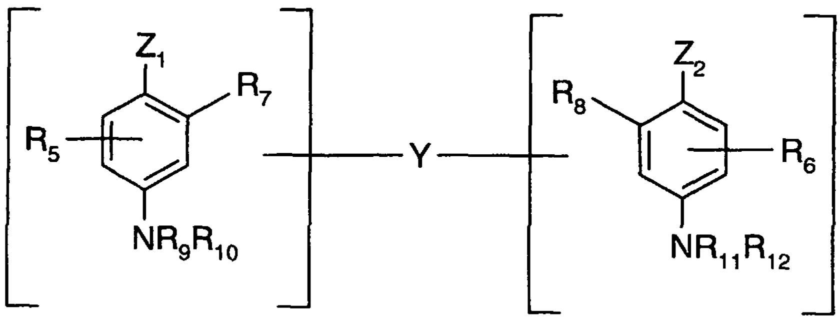

**(IV)**

dans laquelle :

- $Z_1$ et $Z_2$, identiques ou différents, représentent un radical hydroxyle ou $-NH_2$ pouvant être substitué par un radical alkyle en $C_1$-$C_4$ ou par un bras de liaison Y ;
- le bras de liaison Y représente une chaîne alkylène comportant de 1 à 14 atomes de carbone, linéaire ou ramifiée pouvant être interrompue ou terminée par un ou plusieurs groupements azotés et/ou par un ou plusieurs hétéroatomes tels que des atomes d'oxygène, de soufre ou d'azote, et éventuellement substituée par un ou plusieurs radicaux hydroxyle ou alcoxy en $C_1$-$C_6$ ;
- $R_5$ et $R_6$ représentent un atome d'hydrogène ou d'halogène, un radical alkyle en $C_1$-$C_4$, monohydroxyalkyle en $C_1$-$C_4$, polyhydroxyalkyle en $C_2$-$C_4$, aminoalkyle en $C_1$-$C_4$ ou un bras de liaison Y ;
- $R_7$, $R_8$, $R_9$, $R_{10}$, $R_{11}$ et $R_{12}$, identiques ou différents, représentent un atome d'hydrogène, un bras de liaison Y ou un radical alkyle en $C_1$-$C_4$ ; étant entendu que les composés de formule (IV) ne comportent qu'un seul bras de liaison Y par molécule.

Parmi les groupements azotés de la formule (IV) ci-dessus, on peut citer notamment les radicaux amino, monoalkyl($C_1$-$C_4$)amino, dialkyl($C_1$-$C_4$)amino, trialkyl($C_1$-$C_4$)amino, monohydroxyalkyl($C_1$-$C_4$)amino, imidazolinium et ammonium.
Parmi les bases doubles de formules (IV) ci-dessus, on peut plus particulièrement citer le N,N'-bis-(β-hydroxyéthyl)-N,N'-bis-(4'-aminophényl)-1,3-diamino-propanol, la N,N'-bis-(β-hydroxyéthyl)-N,N'-bis-(4'-aminophényl)-éthylènediamine, la N,N'-bis-(4-aminophényl)-tétraméthylènediamine, la N,N'-bis-(β-hydroxyéthyl)-N,N'-bis-(4-aminophényl)-tétraméthylènediamine, la N,N'-bis-(4-méthyl-aminophényl)-tétraméthylènediamine, la N,N'-bis-(éthyl)-N,N'-bis-(4'-amino-3'-méthylphényl)-éthylènediamine, le 1,8-bis-(2,5-diaminophénoxy)-3,5-dioxaoctane, et leurs sels d'addition avec un acide.
Parmi ces bases doubles de formule (IV), le N,N'-bis-(β-hydroxyéthyl)-N,N'-bis-(4'-aminophényl)-1,3-diamino-propanol, le 1,8-bis-(2,5-diaminophénoxy)-3,5-dioxaoctane ou l'un de leurs sels d'addition avec un acide sont particulièrement préférés.

- **(C)** les para-aminophénols répondant à la formule (V) suivante, et leurs sels d'addition avec un acide :

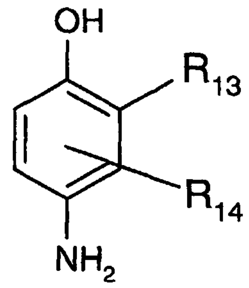

(V)

dans laquelle :

**$R_{13}$** représente un atome d'hydrogène,un atome d'halogène tel que le fluor, un radical alkyle en $C_1$-$C_4$, monohydroxyalkyle en $C_1$-$C_4$, alcoxy($C_1$-$C_4$)alkyle($C_1$-$C_4$) ou aminoalkyle en $C_1$-$C_4$, ou hydroxyalkyl($C_1$-$C_4$)aminoalkyle en $C_1$-$C_4$.

**$R_{14}$** représente un atome d'hydrogène ou un atome d'halogène tel que le fluor, un radical alkyle en $C_1$-$C_4$, monohydroxyalkyle en $C_1$-$C_4$, polyhydroxyalkyle en $C_2$-$C_4$, aminoalkyle en $C_1$-$C_4$, cyanoalkyle en $C_1$-$C_4$ ou alcoxy($C_1$-$C_4$)alkyle($C_1$-$C_4$).

Parmi les para-aminophénols de formule (V) ci-dessus, on peut plus particulièrement citer le para-aminophénol, le 4-amino-3-méthyl-phénol, le 4-amino-3-fluoro-phénol, le 4-amino-3-hydroxyméthyl-phénol, le 4-amino-2-méthylphénol, le 4-amino-2-hydroxyméthyl-phénol, le 4-amino-2-méthoxyméthyl-phénol, le 4-amino-2-aminométhyl-phénol, le 4-amino-2-(β-hydroxyéthyl-aminométhyl)-phénol, et leurs sels d'addition avec un acide.

- **(D)** les ortho-aminophénols utilisables à titre de bases d'oxydation dans le cadre de la présente l'invention, sont notamment choisis parmi le 2-amino-phénol, le 2-amino-1-hydroxy-5-méthyl-benzène, le 2-amino-1-hydroxy-6-méthyl-benzène, le 5-acétamido-2-amino-phénol, et leurs sels d'addition avec un acide.

- **(E)** parmi les bases hétérocycliques utilisables à titre de bases d'oxydation dans les compositions tinctoriales conformes à l'invention, on peut plus particulièrement citer les dérivés pyridiniques, les dérivés pyrimidiniques, les dérivés pyrazoliques, et leurs sels d'addition avec un acide.

Parmi les dérivés pyridiniques, on peut plus particulièrement citer les composés décrits par exemple dans les brevets GB 1 026 978 et GB 1 153 196, comme la 2,5-diamino-pyridine, la 2-(4-méthoxyphényl)amino-3-amino-pyridine, la 2,3-diamino-6-méthoxypyridine, la 2-(β-méthoxyéthyl)amino-3-amino-6-méthoxy pyridine, la 3,4-diamino-pyridine, et leurs sels d'addition avec un acide.

Parmi les dérivés pyrimidiniques, on peut plus particulièrement citer les composés décrits par exemple dans les brevets allemand DE 2 359 399 ou japonais JP 88-169 571 et JP 91-10659 ou demandes de brevet WO 96/15765, comme la 2,4,5,6-tétra-aminopyrimidine, la 4-hydroxy-2,5,6-triaminopyrimidine, la 2-hydroxy-4,5,6-triaminopyrimidine, la 2,4-dihydroxy-5,6-diaminopyrimidine, la 2,5,6-triaminopyrimidine, et les dérivés pyrazolopyrimidiniques tels ceux mentionnés dans la demande de brevet FR-A-2 750 048 et parmi lesquels on peut citer la pyrazolo-[1,5-a]-pyrimidine-3,7-diamine; la 2,5-diméthylpyrazolo-[1,5-a]-pyrimidine-3,7-diamine ; la pyrazolo-[1,5-a]-pyrimidine-3,5-diamine ; la 2,7-diméthyl-pyrazolo-[1,5-a]-pyrimidine-3,5-diamine ; le 3-amino-pyrazolo-[1,5-a]-pyrimidin-7-ol ; le 3-amino-pyrazolo-[1,5-a]-pyrimidin-5-ol; le 2-(3-amino pyrazolo-[1,5-a]-pyrimidin-7-ylamino)-éthanol; le 2-(7-amino-pyrazolo-[1,5-a]-pyrimidin-3-ylamino)-éthanol; le 2-[(3-amino-pyrazolo[1,5-a]pyrimidin-7-yl)-(2-hydroxy-éthyl)-amino]-éthanol; le 2-[(7-amino-pyrazolo[1,5-a]pyrimidin-3-yl)-(2-hydroxy-éthyl)-amino]-éthanol; la 5,6-diméthylpyrazolo-[1,5-a]-pyrimidine-3,7-diamine; la 2,6-diméthyl-pyrazolo-[1,5-a]-pyrimidine-3,7-diamine; la 2, 5, N7, N7-tetraméthyl-pyrazolo-[1,5-a]-pyrimidine-3,7-diamine; la 3-amino-5-méthyl-7-imidazolylpropylamino-pyrazolo-[1,5-a]-pyrimidine; et leurs sels d'addition et leurs formes tautomères, lorsqu'il existe un équilibre tautomérique et leurs sels d'addition avec un acide.

Parmi les dérivés pyrazoliques, on peut plus particulièrement citer les composés décrits dans les brevets DE 3 843 892, DE 4 133 957 et demandes de brevet WO 94/08969, WO 94/08970, FR-A-2 733 749 et DE 195 43 988

comme le 4,5-diamino-1-méthylpyrazole, le 3,4-diamino-pyrazole, le 4,5-diamino-1-(4'-chlorobenzyl)-pyrazole, le 4,5-diamino 1,3-diméthyl-pyrazole, le 4,5-diamino-3-méthyl-1-phényl-pyrazole, le 4,5-diamino 1-méthyl-3-phényl-pyrazole, le 4-amino-1,3-diméthyl-5-hydrazino-pyrazole, le 1-benzyl-4,5-diamino-3-méthyl-pyrazole, le 4,5-diamino-3-tert-butyl-1-méthyl-pyrazole, le 4,5-diamino-1-tert-butyl-3-méthyl-pyrazole, le 4,5-diamino-1-(β-hydroxyéthyl)-3-méthyl pyrazole, le 4,5-diamino-1-(β-hydroxyéthyl)-pyrazole, le 4,5-diamino-1-éthyl-3-méthylpyrazole, le 4,5-diamino-1-éthyl-3-(4'-méthoxyphényl)-pyrazole, le 4,5-diamino-1-éthyl-3-hydroxyméthyl-pyrazole, le 4,5-diamino-3-hydroxyméthyl-1-méthyl-pyrazole, le 4,5-diamino-3-hydroxyméthyl-1-isopropyl-pyrazole, le 4,5-diamino-3-méthyl-1-isopropylpyrazole, le 4-amino-5-(2'-aminoéthyl)amino-1,3-diméthyl-pyrazole, le 3,4,5-triaminopyrazole, le 1-méthyl-3,4,5-triamino-pyrazole, le 3,5-diamino-1-méthyl-4-méthylaminopyrazole, le 3,5-diamino-4-(β-hydroxyéthyl)amino-1-méthyl-pyrazole, et leurs sels d'addition avec un acide.

Selon la présente invention, les bases d'oxydation représentent de préférence de 0,0005 à 12% en poids environ du poids total de la composition et encore plus préférentiellement de 0,005 à 8% en poids environ de ce poids.

Les coupleurs utilisables dans le procédé de teinture selon l'invention sont ceux classiquement utilisés dans les compositions de teinture d'oxydation, c'est-à-dire les méta-aminophénols, les méta-phénylènediamines, les métadiphénols, les naphtols et les coupleurs hétérocycliques tels que par exemple les dérivés indoliques, les dérivés indoliniques, le sésamol et ses dérivés, les dérivés pyridiniques, les dérivés pyrazolotriazoles, les pyrazolones, les indazoles, les benzimidazoles, les benzothiazoles, les benzoxazoles, les 1,3-benzodioxoles, les quinolines et leurs sels d'addition avec un acide.

Ces coupleurs sont plus particulièrement choisis parmi le 2,4-diamino 1-(β-hydroxyéthyloxy)-benzène, le 2-méthyl-5-amino-phénol, le 5-N-(β-hydroxyéthyl)amino-2-méthyl-phénol, le 3-amino-phénol, le 1,3-dihydroxy-benzène, le 1,3-dihydroxy-2-méthyl-benzène, le 4-chloro-1,3-dihydroxy-benzène, le 2-amino 4-(β-hydroxyéthylamino)-1-méthoxy-benzène, le 1,3-diamino-benzène, le 1,3-bis-(2,4-diaminophénoxy)-propane, le sésamol, le 1-amino-2-méthoxy-4,5-méthylènedioxy benzène, l'α-naphtol, le 6-hydroxy-indole, le 4-hydroxy-indole, le 4-hydroxy-N-méthyl indole, la 6-hydroxy-indoline, la 2,6-dihydroxy-4-méthyl-pyridine, le 1-H-3-méthyl-pyrazole-5-one, le 1-phényl-3-méthyl-pyrazole-5-one, la 2-amino-3-hydroxypyridine, le 3,6-diméthyl-pyrazolo-[3,2-c]-1,2,4-triazole, le 2,6-diméthyl-pyrazolo-[1,5-b]-1,2,4-triazole et leurs sels d'addition avec un acide.

Lorsqu'ils sont présents, ces coupleurs représentent de préférence de 0,0001 à 10% en poids environ du poids total de la composition, et encore plus préférentiellement de 0,005 à 5% en poids environ.

D'une manière générale, les sels d'addition avec un acide des bases d'oxydation et coupleurs sont notamment choisis parmi les chlorhydrates, les bromhydrates, les sulfates et les tartrates, les lactates et les acétates.

Colorants directs

Les colorants directs utilisables selon l'invention sont choisis de préférence parmi les colorants directs nitrés benzéniques neutres, acides ou cationiques, les colorants directs azoïques neutres acides ou cationiques, les colorants directs quinoniques et en particulier anthraquinoniques neutres, acides ou cationiques, les colorants directs aziniques, les colorants directs triarylméthaniques, les colorants directs indoaminiques et les colorants directs naturels.

Parmi les colorants directs benzéniques utilisables selon l'invention, on peut citer de manière non limitative les composés suivants:

- 1,4-diamino-2-nitrobenzène
- 1-amino-2 nitre-4-β- hydroxyéthylaminobenzène
- 1-amino-2 nitro-4-bis(β-hydroxyéthyl)-aminobenzène
- 1,4-Bis(β-hydroxyéthylamino)-2-nitrobenzène
- 1-β-hydroxyéthylamino-2-nitro-4-bis-(β-hydroxyéthylamino)-benzène
- 1-β-hydroxyéthylamino-2-nitro-4-aminobenzène
- 1-β-hydroxyéthylamino-2-nitro-4-(éthyl)(β-hydroxyéthyl)-aminobenzène
- 1-amino-3-méthyl-4-β-hydroxyéthylamino-6-nitrobenzène
- 1-amino-2-nitro-4-β-hydroxyéthylamino-5-chlorobenzène
- 1,2-Diamino-4-nitrobenzène
- 1-amino-2-β-hydroxyéthylamino-5-nitrobenzène
- 1,2-Bis-(β-hydroxyéthylamino)-4-nitrobenzène
- 1-amino-2-tris-(hydroxyméthyl)-méthylamino-5-nitrobenzène
- 1-Hydroxy-2-amino-5-nitrobenzène
- 1-Hydroxy-2-amino-4-nitrobenzène
- 1-Hydroxy-3-nitro-4-aminobenzène
- 1-Hydroxy-2-amino-4,6-dinitrobenzène
- 1-β-hydroxyéthyloxy-2-β-hydroxyéthylamino-5-nitrobenzène

- 1-Méthoxy-2-β-hydroxyéthylamino-5-nitrobenzène
- 1-β-hydroxyéthyloxy-3-méthylamino-4-nitrobenzène
- 1-β,γ-dihydroxypropyloxy-3-méthylamino-4-nitrobenzène
- 1-β-hydroxyéthylamino-4-β,γ-dihydroxypropyloxy-2-nitrobenzène
- 1-β,γ-dihydroxypropylamino-4-trifluorométhyl-2-nitrobenzène
- 1-β-hydroxyéthylamino-4-trifluorométhyl-2-nitrobenzène
- 1-β-hydroxyéthylamino-3-méthyl-2-nitrobenzène
- 1-β-aminoéthylamino-5-méthoxy-2-nitrobenzène
- 1-Hydroxy-2-chloro-6-éthylamino-4-nitrobenzène
- 1-Hydroxy-2-chloro-6-amino-4-nitrobenzène
- 1-Hydroxy-6-bis-(β-hydroxyéthyl)-amino-3-nitrobenzène
- 1-β-hydroxyéthylamino-2-nitrobenzène
- 1-Hydroxy-4-β-hydroxyéthylamino-3-nitrobenzène.

Parmi les colorants directs azoïques utilisables selon l'invention on peut citer les colorants azoïques cationiques décrits dans les demandes de brevets WO 95/15144, WO-95/01772 et EP-714954 dont le contenu fait partie intégrante de l'invention.
Parmi ces composés on peut tout particulièrement citer les colorants suivants:

- chlorure de 1,3-diméthyl-2-[[4-(diméthylamino)phényl]azo]-1H-Imidazolium,
- chlorure de 1,3-diméthyl-2-[(4-aminophényl)azo]-1H-Imidazolium,
- méthylsulfate de 1-méthyl-4-[(méthylphénylhydrazono)méthyl]-pyridinium.

On peut également citer parmi les colorants directs azoïques les colorants suivants, décrits dans le COLOUR INDEX INTERNATIONAL 3$^{e}$ édition :

- Disperse Red 17
- Acid Yellow 9
- Acid Black 1
- Basic Red 22
- Basic Red 76
- Basic Yellow 57
- Basic Brown 16
- Acid Yellow 36
- Acid Orange 7
- Acid Red 33
- Acid Red 35
- Basic Brown 17
- Acid Yellow 23
- Acid Orange 24
- Disperse Black 9.

On peut aussi citer le 1-(4'-aminodiphénylazo)-2-méthyl-4bis-(β-hydroxyéthyl) aminobenzène et l'acide 4-hydroxy-3-(2-méthoxyphénylazo)-1-naphtalène sulfonique.
Parmi les colorants directs quinoniques on peut citer les colorants suivants :

- Disperse Red 15
- Solvent Violet 13
- Acid Violet 43
- Disperse Violet 1
- Disperse Violet 4
- Disperse Blue 1
- Disperse Violet 8
- Disperse Blue 3
- Disperse Red 11
- Acid Blue 62
- Disperse Blue 7
- Basic Blue 22

- Disperse Violet 15
- Basic Blue 99

ainsi que les composés suivants :

- 1-N-méthylmorpholiniumpropylamino-4-hydroxyanthraquinone
- 1-Aminopropylamino-4-méthylaminoanthraquinone
- 1-Aminopropylaminoanthraquinone
- 5-β-hydroxyéthyl-1,4-diaminoanthraquinone
- 2-Aminoéthylaminoanthraquinone
- 1,4-Bis-(β,γ-dihydroxypropylamino)-anthraquinone.

Parmi les colorants aziniques on peut citer les composés suivants :

- Basic Blue 17
- Basic Red 2.

Parmi les colorants triarylméthaniques utilisables selon l'invention, on peut citer les composés suivants :

- Basic Green 1
- Acid blue 9
- Basic Violet 3
- Basic Violet 14
- Basic Blue 7
- Acid Violet 49
- Basic Blue 26
- Acid Blue 7

Parmi les colorants indoaminiques utilisables selon l'invention, on peut citer les composés suivants :

- 2-β-hydroxyéthylamino-5-[bis-(β-4'-hydroxyéthyl)amino]anilino-1,4-benzoquinone
- 2-β-hydroxyéthylamino-5-(2'-méthoxy-4'-amino)anilino-1,4-benzoquinone
- 3-N(2'-Chloro-4'-hydroxy)phényl-acétylamino-6-méthoxy-1,4-benzoquinone imine
- 3-N(3'-Chloro-4'-méthylamino)phényl-uréido-6-méthyl-1,4-benzoquinone imine
- 3-[4'-N-(Ethyl,carbamylméthyl)-amino]-phényl-uréido-6-méthyl-1,4-benzoquinone imine

Parmi les colorants directs naturels utilisables selon l'invention, on peut citer la lawsone, la juglone, l'alizarine, la purpurine, l'acide carminique, l'acide kermésique, la purpurogalline, le protocatéchaldéhyde, l'indigo, l'isatine, la curcumine, la spinulosine, l'apigénidine. On peut également utiliser les extraits ou décoctions contenant ces colorants naturels et notamment les cataplasmes ou extraits à base de henné.
Le ou les colorants directs représentent de préférence de 0,001 à 20% en poids environ du poids total de la composition prête à l'emploi et encore plus préférentiellement de 0,005 à 10% en poids environ.
Les compositions de l'invention comprennent de préférence un ou plusieurs tensioactifs. Le ou les tensioactifs peuvent être indifféremment choisis, seuls ou en mélanges, au sein des tensioactifs anioniques, amphotères, non ioniques, zwittérioniques et cationiques.
Les tensioactifs convenant à la mise en oeuvre de la présente invention sont notamment les suivants :

(i) Tensioactif(s) anioniaue(s) :

A titre d'exemple de tensio-actifs anioniques utilisables, seuls ou mélanges, dans le cadre de la présente invention, on peut citer notamment (liste non limitative) les sels (en particulier sels alcalins, notamment de sodium, sels d'ammonium, sels d'amines, sels d'aminoalcools ou sels de magnésium) des composés suivants : les alkylsulfates, les alkyléthersulfates, alkylamidoéthersulfates, alkylarylpolyéthersulfates, monoglycérides sulfates ; les alkylsulfonates, alkylphosphates, alkylamidesulfonates, alkylarylsulfonates, α-oléfine-sulfonates, paraffine-sulfonates ; les alkyl($C_6$-$C_{24}$) sulfosuccinates, les alkyl($C_6$-$C_{24}$) éthersulfosuccinates, les alkyl($C_6$-$C_{24}$) amidesulfosuccinates ; les alkyl($C_6$-$C_{24}$) sulfoacétates ; les acyl($C_6$-$C_{24}$) sarcosinates et les acyl($C_6$-$C_{24}$) glutamates. On peut également utiliser les esters d'alkyl ($C_6$-$C_{24}$)polyglycosides carboxyliques tels que les alkylglucoside citrates, les alkylpolyglycoside tartrate et les alkylpolyglycoside sulfosuccinates., les alkylsulfosuccinamates ; les acyliséthionates et les N-acyltaurates, le radical alkyle ou

acyle de tous ces différents composés comportant de préférence de 12 à 20 atomes de carbone, et le radical aryl désignant de préférence un groupement phényle ou benzyle. Parmi les tensioactifs anioniques encore utilisables, on peut également citer les sels d'acides gras tels que les sels des acides oléique, ricinoléique, palmitique, stéarique, les acides d'huile de coprah ou d'huile de coprah hydrogénée ; les acyl-lactylates dont le radical acyle comporte 8 à 20 atomes de carbone. On peut également utiliser les acides d'alkyl D galactoside uroniques et leurs sels, les acides alkyl ($C_6$-$C_{24}$) éther carboxyliques polyoxyalkylénés, les acides alkyl($C_6$-$C_{24}$)aryl éther carboxyliques polyoxyalkylénés, les acides alkyl($C_6$-$C_{24}$) amido éther carboxyliques polyoxyalkylénés et leurs sels, en particulier ceux comportant de 2 à 50 groupements oxyde d'alkylène en particulier d'éthylène, et leurs mélanges.

(ii) Tensioactif(s) non ionigue(s) :

Les agents tensioactifs non-ioniques sont, eux aussi, des composés bien connus en soi (voir notamment à cet égard "Handbook of Surfactants" par M.R. PORTER, éditions Blackie & Son (Glasgow and London), 1991, pp 116-178) et leur nature ne revet pas, dans le cadre de la présente invention, de caractère critique. Ainsi, ils peuvent être notamment choisis parmi (liste non limitative) les alcools, les alpha-diols, les alkylphénols polyéthoxylés, polypropoxylés, ayant une chaîne grasse comportant par exemple 8 à 18 atomes de carbone, le nombre de groupements oxyde d'éthylène ou oxyde de propylène pouvant aller notamment de 2 à 50. On peut également citer les copolymères d'oxyde d'éthylène et de propylène, les condensats d'oxyde d'éthylène et de propylène sur des alcools gras ; les amides gras polyéthoxylés ayant de préférence de 2 à 30 moles d'oxyde d'éthylène, les amides gras polyglycérolés comportant en moyenne 1 à 5 groupements glycérol et en particulier 1,5 à 4 ; les amines grasses polyéthoxylées ayant de préférence 2 à 30 moles d'oxyde d'éthylène ; les esters d'acides gras du sorbitan oxyéthylénés ayant de 2 à 30 moles d'oxyde d'éthylène ; les esters d'acides gras du sucrose, les esters d'acides gras du polyéthylèneglycol, les alkylpolyglycosides, les dérivés de N-alkyl glucamine, les oxydes d'amines tels que les oxydes d'alkyl ($C_{10}$ - $C_{14}$) amines ou les oxydes de N-acylaminopropylmorpholine.

(iii) Tensioactif(s) amphotère(s) ou zwittérionique(s) :

Les agents tensioactifs amphotères ou zwitterioniques, dont la nature ne revet pas dans le cadre de la présente invention de caractère critique, peuvent être notamment (liste non limitative) des dérivés d'amines secondaires ou tertiaires aliphatiques, dans lesquels le radical aliphatique est une chaîne linéaire ou ramifiée comportant 8 à 18 atomes de carbone et contenant au moins un groupe anionique hydrosolubilisant (par exemple carboxylate, sulfonate, sulfate, phosphate ou phosphonate) ; on peut citer encore les alkyl ($C_8$-$C_{20}$) bétaïnes, les sulfobétaïnes, les alkyl ($C_8$-$C_{20}$) amidoalkyl ($C_1$-$C_6$) betaïnes ou les alkyl ($C_8$-$C_{20}$) amidoalkyl ($C_1$-$C_6$) sulfobétaïnes.

Parmi les dérivés d'amines, on peut citer les produits vendus sous la dénomination MIRANOL, tels que décrits dans les brevets US-2 528 378 et US-2 781 354 et classés dans le dictionnaire CTFA, 3ème édition, 1982, sous les dénominations Amphocarboxyglycinates et Amphocarboxypropionates de structures respectives :

$$R_2\text{-CONHCH}_2\text{CH}_2\text{-N}(R_3)(R_4)(\text{CH}_2\text{COO}^-)$$

dans laquelle : $R_2$ désigne un radical alkyle d'un acide $R_2$-COOH présent dans l'huile de coprah hydrolysée, un radical heptyle, nonyle ou undécyle, $R_3$ désigne un groupement bêta-hydroxyéthyle et $R_4$ un groupement carboxyméthyle ; et

$$R_2'\text{-CONHCH}_2\text{CH}_2\text{-N(B)(C)}$$

dans laquelle :

B représente $-CH_2CH_2OX'$, C représente $-(CH_2)_Z-Y'$, avec z = 1 ou 2,
X' désigne le groupement $-CH_2CH_2-COOH$ ou un atome d'hydrogène
Y' désigne -COOH ou le radical $-CH_2-$ CHOH - $SO_3H$
$R_2'$ désigne un radical alkyle d'un acide $R_9$ -COOH présent dans l'huile de coprah ou dans l'huile de lin hydrolysée, un radical alkyle, notamment en $C_7$, $C_9$, $C_{11}$ ou $C_{13}$, un radical alkyle en $C_{17}$ et sa forme iso, un radical $C_{17}$ insaturé.

Ces composés sont classés dans le dictionnaire CTFA, Sème édition, 1993, sous les dénominations Disodium Cocoamphodiacetate, Disodium Lauroamphodiacetate, Disodium Caprylamphodiacetate, Disodium Capryloamphodiacetate, Disodium Cocoamphodipropionate, Disodium Lauroamphodipropionate, Disodium Caprylamphodipropionate, Disodium Capryloamphodipropionate, Lauroamphodipropionic acid, Cocoamphodipropionic acid.
A titre d'exemple on peut citer le cocoamphodiacetate commercialisé sous la dénomination commerciale MIRANOL®

C2M concentré par la société RHODIA CHIMIE.

(iv) Tensioactifs cationiques :

Parmi les tensioactifs cationiques on peut citer en particulier (liste non limitative) : les sels d'amines grasses primaires, secondaires ou tertiaires, éventuellement polyoxyalkylénées ; les sels d'ammonium quaternaire tels que les chlorures ou les bromures de tétraalkylammonium, d'alkylamidoalkyltrialkylammonium, de trialkylbenzylammonium, de trialkylhydroxyalkyl-ammonium ou d'alkylpyridinium; les dérivés d'imidazoline ; ou les oxydes d'amines à caractère cationique.

Les quantités d'agents tensioactifs présents dans la composition selon l'invention peuvent varier de 0,01 à 40% et de préférence de 0,5 à 30% du poids total de la composition.

Milieu

Le milieu de la composition cosmétiquement acceptable, est de préférence un milieu aqueux constitué par de l'eau et peut avantageusement contenir des solvants organiques acceptables sur le plan cosmétique, dont plus particulièrement, des alcools tels que l'alcool éthylique, l'alcool isopropylique, l'alcool benzylique, et l'alcool phényléthylique, ou des glycols ou éthers de glycol tels que, par exemple, les éthers monométhylique, monoéthylique et monobutylique d'éthylèneglycol, le propylèneglycol ou ses éthers tels que, par exemple, le monométhyléther de propylèneglycol, le butylèneglycol, le dipropylèneglycol ainsi que les alkyléthers de diéthylèneglycol comme par exemple, le monoéthyléther ou le monobutyléther du diéthylèneglycol. Les solvants peuvent alors être présents dans des concentrations comprises entre environ 0,5 et 20% et, de préférence, entre environ 2 et 10% en poids par rapport au poids total de la composition.

Agents ingrédients

Les compositions selon l'invention peuvent également comprendre des agents d'ajustement de la rhéologie tels que les épaississants cellulosiques (hydroxyéthylcellulose, hydroxypropylcellulose, carboxyméthylcellulose..), la gomme de guar et ses dérivés( hydroxypropylguar..), les gommes d'origine microbienne (gomme de xanthane, gomme de scléroglucane..), les épaississants synthétiques tels que les homopolymères réticulés d'acide acrylique ou d'acide acrylamidopropanesulfonique.

Des compositions préférées selon la présente invention comprennent en outre au moins un polymère associatif ionique ou non ionique choisi par exemple parmi les polymères commercialisés sous les appellations PEMULEN ®TR1 ou TR2 par la société GOODRICH, SALCARE SC90® par la société ALLIED COLLOIDS, ACULYN® 22, 28, 33, 44, ou 46 par la société ROHM & HAAS et ELFACOS® T210 et T212 par la société AKZO, dans la proportion de 0,01 à 10% en poids du poids total de la composition.

D'autres compositions préférées selon la présente invention comprennent en outre au moins un polymère conditionneur cationique ou amphotère bien connu de la technique dans le domaine de la teinture des fibres kératiniques humaines dans la proportion de 0,01 à 10% en poids par rapport au poids total de la composition, de préférence de 0,05 à 5% et plus préférentiellement encore de 0,1 à 3%.

Polymères cationiques

Au sens de la présente invention, l'expression "polymère cationique" désigne tout polymère contenant des groupements cationiques et/ou des groupements ionisables en groupements cationiques.

Les polymères cationiques utilisables conformément à la présente invention peuvent être choisis parmi tous ceux déja connus en soi comme améliorant les propriétés cosmétiques des cheveux, à savoir notamment ceux décrits dans la demande de brevet EP-A-337 354 et dans les brevets français FR-2 270 846, 2 383 660, 2 598 611, 2 470 596 et 2 519 863.

Les polymères cationiques préférés sont choisis parmi ceux qui contiennent des motifs comportant des groupements amine primaire, secondaire, tertiaire et/ou quaternaire pouvant, soit faire partie de la chaîne principale polymère, soit être portés par un substituant latéral directement relié à celle-ci.

Les polymères cationiques utilisés ont généralement une masse moléculaire moyenne en nombre comprise entre 500 et $5.10^6$ environ, et de préférence comprise entre $10^3$ et $3.10^6$ environ.

Parmi les polymères cationiques, on peut citer plus particulièrement les polymères du type polyamine, polyaminoamide et polyammonium quaternaire.

Ce sont des produits connus. Ils sont notamment décrits dans les brevets français n° 2 505 348 ou 2 542 997. Parmi lesdits polymères, on peut citer :

**(1)** Les homopolymères ou copolymères dérivés d'esters ou d'amides acryliques ou méthacryliques et comportant au moins un des motifs de formules (I), (II), (III) ou (IV) suivantes:

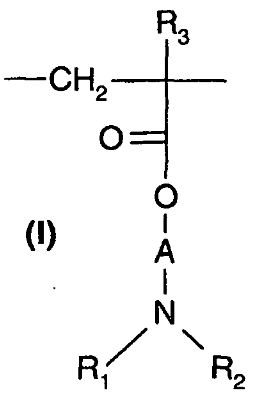
(I)

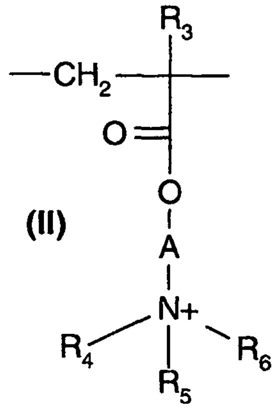
(II)

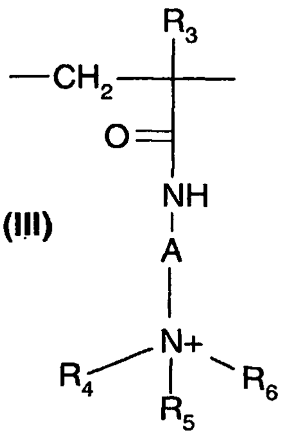
(III)

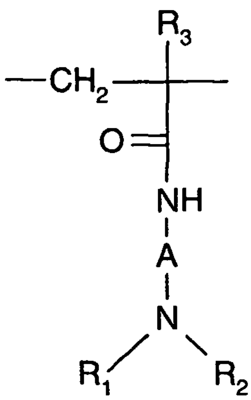
(IV)

dans lesquelles:

$R_3$, identiques ou différents, désignent un atome d'hydrogène ou un radical $CH_3$;
A, identiques ou différents, représentent un groupe alkyle, linéaire ou ramifié, de 1 à 6 atomes de carbone, de préférence 2 ou 3 atomes de carbone ou un groupe hydroxyalkyle de 1 à 4 atomes de carbone ;
$R_4$, $R_5$, $R_6$, identiques ou différents, représentent un groupe alkyle ayant de 1 à 18 atomes de carbone ou un radical benzyle et de préférence un groupe alkyle ayant de 1 à 6 atomes de carbone;
$R_1$ et $R_2$, identiques ou différents, représentent hydrogène ou un groupe alkyle ayant de 1 à 6 atomes de carbone et de préférence méthyle ou éthyle;
X désigne un anion dérivé d'un acide minéral ou organique tel qu'un anion méthosulfate ou un halogénure tel que chlorure ou bromure.

Les polymères de la famille (1) peuvent contenir en outre un ou plusieurs motifs dérivant de comonomères pouvant être choisis dans la famille des acrylamides, méthacrylamides, diacétones acrylamides, acrylamides et méthacrylamides substitués sur l'azote par des alkyles inférieurs ($C_1$-$C_4$), des acides acryliques ou méthacryliques ou leurs esters, des vinyllactames tels que la vinylpyrrolidone ou le vinylcaprolactame, des esters vinyliques.
Ainsi, parmi ces polymères de la famille (1), on peut citer :

- les copolymères d'acrylamide et de diméthylaminoéthyl méthacrylate quaternisé au sulfate de diméthyle ou avec un halogénure de diméthyle, tel que celui vendu sous la dénomination HERCOFLOC par la société HERCULES,

- les copolymères d'acrylamide et de chlorure de méthacryloyloxyéthyltriméthylammonium décrits par exemple dans la demande de brevet EP-A-080976 et vendus sous la dénomination BINA QUAT P 100 par la société CIBA GEIGY,
- le copolymère d'acrylamide et de méthosulfate de méthacryloyloxyéthyltriméthylammonium vendu sous la dénomination RETEN par la société HERCULES,
- les copolymères vinylpyrrolidone / acrylate ou méthacrylate de dialkylaminoalkyle quaternisés ou non, tels que les produits vendus sous la dénomination "GAFQUAT" par la société ISP comme par exemple "GAFQUAT 734" ou "GAFQUAT 755" ou bien les produits dénommés "COPOLYMER 845, 958 et 937". Ces polymères sont décrits en détail dans les brevets français 2.077.143 et 2.393.573,
- les terpolymères méthacrylate de diméthyl amino éthyle/ vinylcaprolactame/ vinylpyrrolidone tel que le produit vendu sous la dénomination GAFFIX VC 713 par la société ISP,
- les copolymère vinylpyrrolidone / méthacrylamidopropyl dimethylamine commercialisés notamment sous la dénomination STYLEZE CC 10 par ISP,
- et les copolymères vinylpyrrolidone / méthacrylamide de diméthylaminopropyle quaternisés tel que le produit vendu sous la dénomination "GAFQUAT HS 100" par la société ISP.

**(2)** Les dérivés d'éthers de cellulose comportant des groupements ammonium quaternaire décrits dans le brevet français 1 492 597, et en particulier les polymères commercialisés sous les dénominations "JR" (JR 400, JR 125, JR 30M) ou "LR" (LR 400, LR 30M) par la Société Union Carbide Corporation. Ces polymères sont également définis dans le dictionnaire CTFA comme des ammonium quaternaires d'hydroxyéthylcellulose ayant réagi avec un époxyde substitué par un groupement triméthylammonium .

**(3)** Les dérivés de cellulose cationiques tels que les copolymères de cellulose ou les dérivés de cellulose greffés avec un monomère hydrosoluble d'ammonium quaternaire, et décrits notamment dans le brevet US 4 131 576, tels que les hydroxyalkyl celluloses, comme les hydroxyméthyl-, hydroxyéthyl- ou hydroxypropyl celluloses greffées notamment avec un sel de méthacryloyléthyl triméthylammonium, de méthacrylmidopropyl triméthylammonium, de diméthyl-diallylammonium.
Les produits commercialisés répondant à cette définition sont plus particulièrement les produits vendus sous la dénomination "Celquat L 200" et "Celquat H 100" par la Société National Starch.

**(4)** Les polysaccharides cationiques décrits plus particulièrement dans les brevets US 3 589 578 et 4 031 307 tel que les gommes de guar contenant des groupements cationiques trialkylammonium. On utilise par exemple des gommes de guar modifiées par un sel (par ex. chlorure) de 2,3-époxypropyl triméthylammonium.
De tels produits sont commercialisés notamment sous les dénominations commerciales de JAGUAR C13 S, JAGUAR C 15, JAGUAR C 17 ou JAGUAR C162 par la société MEYHALL.

**(5)** Les polymères constitués de motifs pipérazinyle et de radicaux divalents alkylène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quaternisation de ces polymères. De tels polymères sont notamment décrits dans les brevets français 2.162.025 et 2.280.361.

**(6)** Les polyaminoamides solubles dans l'eau préparés en particulier par polycondensation d'un composé acide avec une polyamine ; ces polyaminoamides peuvent être réticulés par une épihalohydrine, un diépoxyde, un dianhydride, un dianhydride non saturé, un dérivé bis-insaturé, une bis-halohydrine, un bis-azétidinium, une bis-haloacyldiamine, un bis-halogénure d'alkyle ou encore par un oligomère résultant de la réaction d'un composé bifonctionnel réactif vis-à-vis d'une bis-halohydrine, d'un bis-azétidinium, d'une bis-haloacyldiamine, d'un bis-halogénure d'alkyle, d'une épilhalohydrine, d'un diépoxyde ou d'un dérivé bis-insaturé ; l'agent réticulant étant utilisé dans des proportions allant de 0,025 à 0,35 mole par groupement amine du polyaminoamide ; ces polyaminoamides peuvent être alcoylés ou s'ils comportent une ou plusieurs fonctions amines tertiaires, quaternisées. De tels polymères sont notamment décrits dans les brevets français 2.252.840 et 2.368.508 .

(7) Les dérivés de polyaminoamides résultant de la condensation de polyalcoylènes polyamines avec des acides polycarboxyliques suivie d'une alcoylation par des agents bifonctionnels. On peut citer par exemple les polymères acide adipique-diacoylaminohydroxyalcoyldialoylène triamine dans lesquels le radical alcoyle comporte de 1 à 4 atomes de carbone et désigne de préférence méthyle, éthyle, propyle. De tels polymères sont notamment décrits dans le brevet français 1.583.363.
Parmi ces dérivés, on peut citer plus particulièrement les polymères acide adipique/diméthylaminohydroxypropyl/diéthylène triamine vendus sous la dénomination "Cartaretine F, F4 ou F8" par la société Sandoz.

**(8)** Les polymères obtenus par réaction d'une polyalkylène polyamine comportant deux groupements amine primaire et au moins un groupement amine secondaire avec un acide dicarboxylique choisi parmi l'acide diglycolique et les acides dicarboxyliques aliphatiques saturés ayant de 3 à 8 atomes de carbone. Le rapport molaire entre le polyalkylène polylamine et l'acide dicarboxylique étant compris entre 0,8 : 1 et 1,4 : 1; le polyaminoamide en résultant étant amené à réagir avec l'épichlorhydrine dans un rapport molaire d'épichlorhydrine par rapport au groupement amine secondaire du polyaminoamide compris entre 0,5 : 1 et 1,8 : 1. De tels polymères sont notamment décrits dans les brevets américains 3.227.615 et 2.961.347.
Des polymères de ce type sont en particulier commercialisés sous la dénomination "Hercosett 57" par la société Hercules Inc. ou bien sous la dénomination de "PD 170" ou "Delsette 101" par la société Hercules dans le cas du copolymère d'acide adipique/époxypropyl/diéthylène-triamine.

**(9)** Les cyclopolymères d'alkyl diallyl amine ou de dialkyl diallyl ammonium tels que les homopolymères ou copolymères comportant comme constituant principal de la chaîne des motifs répondant aux formules (V) ou (VI) :

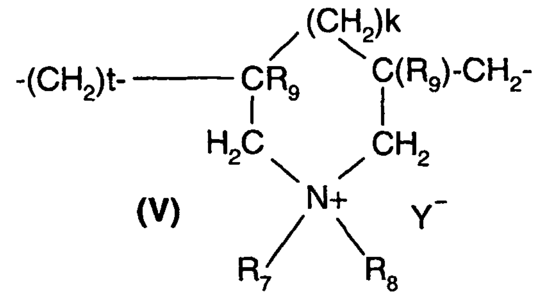
(V)

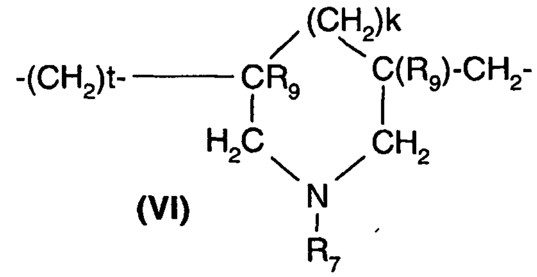
(VI)

formules dans lesquelles k et t sont égaux à 0 ou 1, la somme k + t étant égale à 1 ; $R_9$ désigne un atome d'hydrogène ou un radical méthyle ; $R_7$ et $R_8$, indépendamment l'un de l'autre, désignent un groupement alkyle ayant de 1 à 8 atomes de carbone, un groupement hydroxyalkyle dans lequel le groupement alkyle a de préférence 1 à 5 atomes de carbone, un groupement amidoalkyle inférieur ($C_1$-$C_4$), ou $R_7$ et $R_8$ peuvent désigner conjointement avec l'atome d'azote auquel ils sont rattachés, des groupement hétérocycliques, tels que pipéridinyle ou morpholinyle ; $R_7$ et $R_8$ indépendamment l'un de l'autre désignent de préférence un groupement alkyle ayant de 1 à 4 atomes de carbone ; $Y^-$ est un anion tel que bromure, chlorure, acétate, borate, citrate, tartrate, bisulfate, bisulfite, sulfate, phosphate.
Ces polymères sont notamment décrits dans le brevet français 2.080.759 et dans son certificat d'addition 2.190.406.
Parmi les polymères définis ci-dessus, on peut citer plus particulièrement l'homopolymère de chlorure de diméthyldiallylammonium vendu sous la dénomination "Merquat 100" par la société Calgon (et ses homologues de faible masse moléculaire moyenne en poids) et les copolymères de chlorure de diallyldiméthylammonium et d'acrylamide commercialisés sous la dénomination "MERQUAT 550".

**(10)** Le polymère de diammonium quaternaire contenant des motifs récurrents répondant à la formule :

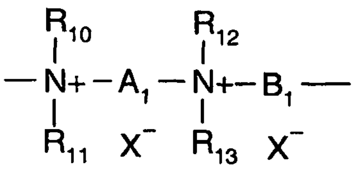
**(VII)**

formule (VII) dans laquelle :

$R_{10}$, $R_{11}$, $R_{12}$ et $R_{13}$, identiques ou différents, représentent des radicaux aliphatiques, alicycliques, ou arylaliphatiques contenant de 1 à 20 atomes de carbone ou des radicaux hydroxyalkylaliphatiques inférieurs, ou bien $R_{10}$, $R_{11}$, $R_{12}$ et $R_{13}$, ensemble ou séparément, constituent avec les atomes d'azote auxquels ils sont rattachés des hétérocycles contenant éventuellement un second hétéroatome autre que l'azote ou bien $R_{10}$, $R_{11}$, $R_{12}$ et $R_{13}$ représentent un radical alkyle en $C_1$-$C_6$ linéaire ou ramifié substitué par un groupement nitrile, ester, acyle, amide ou -CO-O- $R_{14}$-D ou -CO-NH-$R_{14}$-D où $R_{14}$ est un alkylène et D un groupement ammonium quaternaire ;
$A_1$ et $B_1$ représentent des groupements polyméthyléniques contenant de 2 à 20 atomes de carbone pouvant être linéaires ou ramifiés, saturés ou insaturés, et pouvant contenir, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et $X^-$ désigne un anion dérivé d'un acide minéral ou organique;
$A_1$, $R_{10}$ et $R_{12}$ peuvent former avec les deux atomes d'azote auxquels ils sont rattachés un cycle pipérazinique ; en outre si $A_1$ désigne un radical alkylène ou hydroxyalkylène linéaire ou ramifié, saturé ou insaturé, $B_1$ peut également désigner un groupement - $(CH_2)n$-CO-D-OC-$(CH_2)n$- - dans lequel n est compris entre 1 et 100 et de préférence entre 1 et 50, et D désigne :

a) un reste de glycol de formule : -O-Z-O-, où Z désigne un radical hydrocarboné linéaire ou ramifié ou un groupement répondant à l'une des formules suivantes :

$$-(CH_2-CH_2-O)x-CH_2-CH_2-$$

$$-[CH_2-CH(CH_3)-O]y-CH_2-CH(CH_3)-$$

où x et y désignent un nombre entier de 1 à 4, représentant un degré de polymérisation défini et unique ou un nombre quelconque de 1 à 4 représentant un degré de polymérisation moyen ;
b) un reste de diamine bis-secondaire tel qu'un dérivé de pipérazine ;
c) un reste de diamine bis-primaire de formule : -NH-Y-NH-, où Y désigne un radical hydrocarboné linéaire ou ramifié, ou bien le radical bivalent

$$-CH_2-CH_2-S-S-CH_2-CH_2- ;$$

d) un groupement uréylène de formule : -NH-CO-NH-.

De préférence, $X^-$ est un anion tel que le chlorure ou le bromure.
Ces polymères ont une masse moléculaire moyenne en nombre généralement comprise entre 1000 et 100000.
Des polymères de ce type sont notamment décrits dans les brevets français 2.320.330, 2.270.846, 2.316.271, 2.336.434 et 2.413.907 et les brevets US 2.273.780, 2.375.853, 2.388.614, 2.454.547, 3.206.462, 2.261.002, 2.271.378, 3.874.870, 4.001.432, 3.929.990, 3.966.904, 4.005.193, 4.025.617, 4.025.627, 4.025.653, 4.026.945 et 4.027.020.
On peut utiliser plus particulièrement les polymères qui sont constitués de motifs récurrents répondant à la formule (VIII) suivante:

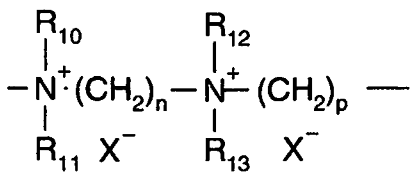

**(VIII)**

dans laquelle $R_{10}$, $R_{11}$, $R_{12}$ et $R_{13}$, identiques ou différents, désignent un radical alkyle ou hydroxyalkyle ayant de 1 à 4 atomes de carbone environ, n et p sont des nombres entiers variant de 2 à 20 environ et, $X^-$ est un anion dérivé d'un acide minéral ou organique.

**(11)** Les polymères de polyammonium quaternaire constitués de motifs récurrents de formule (IX):

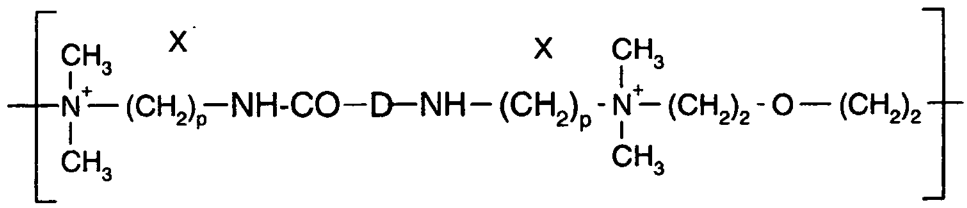

**(IX)**

dans laquelle p désigne un nombre entier variant de 1 à 6 environ, D peut être nul ou peut représenter un groupement $-(CH_2)_r$ -CO- dans lequel r désigne un nombre égal à 4 ou à 7, $X^-$ est un anion;

De tels polymères peuvent être préparés selon les procédés décrits dans les brevets U.S.A. n° 4 157 388, 4 702 906, 4 719 282. Ils sont notamment décrits dans la demande de brevet EP-A-122 324.

Parmi eux, on peut par exemple citer, les produits "Mirapol A 15", "Mirapol AD1", "Mirapol AZ1" et "Mirapol 175" vendus par la société Miranol.

**(12)** Les polymères quaternaires de vinylpyrrolidone et de vinylimidazole tels que par exemple les produits commercialisés sous les dénominations Luviquat FC 905, FC 550 et FC 370 par la société B.A.S.F.

**(13)** Les polyamines comme le Polyquart H vendu par HENKEL, référencé sous le nom de " POLYETHYLENEGLYCOL (15) TALLOW POLYAMINE " dans le dictionnaire CTFA.

**(14)** Les polymères réticulés de sels de méthacryloyloxyalkyl($C_1$-$C_4$) trialkyl($C_1$-$C_4$)ammonium tels que les polymères obtenus par homopolymérisation du diméthylaminoéthylméthacrylate quaternisé par le chlorure de méthyle, ou par copolymérisation de l'acrylamide avec le diméthylaminoéthylméthacrylate quaternisé par le chlorure de méthyle, l'homo ou la copolymérisation étant suivie d'une réticulation par un composé à insaturation oléfinique, en particulier le méthylène bis acrylamide. On peut plus particulièrement utiliser un copolymère réticulé acrylamide/chlorure de méthacryloyloxyéthyl triméthylammonium (20/80 en poids) sous forme de dispersion contenant 50 % en poids dudit copolymère dans de l'huile minérale. Cette dispersion est commercialisée sous le nom de "SALCARE® SC 92" par la Société ALLIED COLLOIDS. On peut également utiliser un homopolymère réticulé du chlorure de méthacryloyloxyéthyl triméthylammonium contenant environ 50 % en poids de l'homopolymère dans de l'huile minérale ou dans un ester liquide. Ces dispersions sont commercialisées sous les noms de " SALCARE® SC 95 " et "SALCARE® SC 96 " par la Société ALLIED COLLOIDS.

D'autres polymères cationiques utilisables dans le cadre de l'invention sont des polyalkylèneimines, en particulier des polyéthylèneimines, des polymères contenant des motifs vinylpyridine ou vinylpyridinium, des condensats de polyamines et d'épichlorhydrine, des polyuréylènes quaternaires et les dérivés de la chitine.

Parmi tous les polymères cationiques susceptibles d'être utilisés dans le cadre de la présente invention, on préfère mettre en oeuvre les polymères des familles (1), (9), (10) (11) et (14) et encore plus préférentiellement les polymères aux motifs récurrents de formules (W) et (U) suivantes :

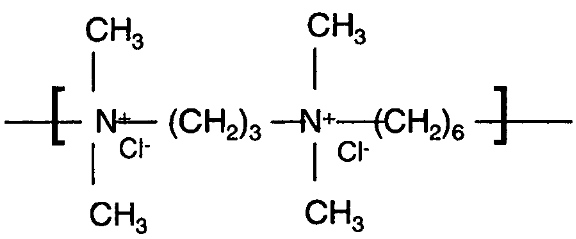

**(W)**

et notamment ceux dont le poids moléculaire, déterminé par chromatographie par perméation de gel, est compris entre 9500 et 9900;

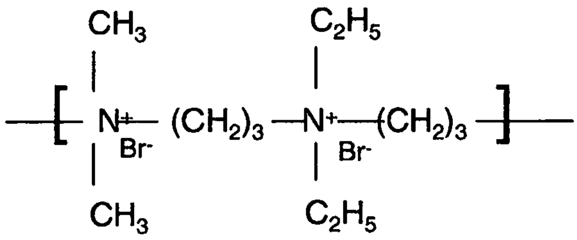

(U)

et notamment ceux dont le poids moléculaire, déterminé par chromatographie par perméation de gel, est d'environ 1200.

Polymères amphotères

Les polymères amphotères utilisables conformément à la présente invention peuvent être choisis parmi les polymères comportant des motifs K et M répartis statistiquement dans la chaîne polymère, où K désigne un motif dérivant d'un monomère comportant au moins un atome d'azote basique et M désigne un motif dérivant d'un monomère acide comportant un ou plusieurs groupements carboxyliques ou sulfoniques, ou bien K et M peuvent désigner des groupements dérivant de monomères zwittérioniques de carboxybétaïnes ou de sulfobétaïnes;
K et M peuvent également désigner une chaîne polymère cationique comportant des groupements amine primaire, secondaire, tertiaire ou quaternaire, dans laquelle au moins l'un des groupements amine porte un groupement carboxylique ou sulfonique relié par l'intermédiaire d'un radical hydrocarboné, ou bien K et M font partie d'une chaîne d'un polymère à motif éthylène $\alpha,\beta$-dicarboxylique dont l'un des groupements carboxyliques a été amené à réagir avec une polyamine comportant un ou plusieurs groupements amine primaire ou secondaire.

Les polymères amphotères répondant à la définition donnée ci-dessus plus particulièrement préférés sont choisis parmi les polymères suivants :

(1) Les polymères résultant de la copolymérisation d'un monomère dérivé d'un composé vinylique portant un groupement carboxylique tel que plus particulièrement l'acide acrylique, l'acide méthacrylique, l'acide maléique, l'acide alpha-chloracrylique, et d'un monomère basique dérivé d'un composé vinylique substitué contenant au moins un atome basique tel que plus particulièrement les dialkylaminoalkylméthacrylate et acrylate, les dialkylaminoalkylméthacrylamide et acrylamide. De tels composés sont décrits dans le brevet américain n° 3 836 537. On peut également citer le copolymère acrylate de sodium / chlorure d'acrylamidopropyl trimethyl ammonium vendu sous la dénomination POLYQUART KE 3033 par la Société HENKEL.
Le composé vinylique peut être également un sel de dialkyldiallylammonium tel que le chlorure de diméthyldiallylammonium. Les copolymères d'acide acrylique et de ce dernier monomère sont proposés sous les appellations MERQUAT 280, MERQUAT 295 et MERQUAT PLUS 3330 par la société CALGON.

(2) Les polymères comportant des motifs dérivant :

a) d'au moins un monomère choisi parmi les acrylamides ou les méthacrylamides substitués sur l'azote par un radical alkyle,
b) d'au moins un comonomère acide contenant un ou plusieurs groupements carboxyliques réactifs, et
c) d'au moins un comonomère basique tel que des esters à substituants amine primaire, secondaire, tertiaire et quaternaire des acides acrylique et méthacrylique et le produit de quaternisation du méthacrylate de diméthylaminoéthyle avec le sulfate de diméthyle ou diéthyle.

Les acrylamides ou méthacrylamides N-substitués plus particulièrement préférés selon l'invention sont les groupements dont les radicaux alkyle contiennent de 2 à 12 atomes de carbone et plus particulièrement le N-éthylacrylamide, le N-tertiobutyl-acrylamide, le N-tertiooctyl-acrylamide, le N-octylacrylamide, le N-décylacrylamide, le N-dodécylacrylamide ainsi que les méthacrylamides correspondants.
Les comonomères acides sont choisis plus particulièrement parmi les acides acrylique, méthacrylique, crotonique, itaconique, maléique, fumarique ainsi que les monoesters d'alkyle ayant 1 à 4 atomes de carbone des acides ou des anhydrides maléique ou fumarique.
Les comonomères basiques préférés sont des méthacrylates d'aminoéthyle, de butyl aminoéthyle, de N,N'-diméthylaminoéthyle, de N-tertio-butylaminoéthyle.
On utilise particulièrement les copolymères dont la dénomination CTFA (4ème Ed., 1991) est Octylacrylamide/acrylates/butylaminoethylmethacrylate copolymer tels que les produits vendus sous la dénomination AMPHOMER ou LOVOCRYL 47 par la société NATIONAL STARCH.

(3) Les polyaminoamides réticulés et alcoylés partiellement ou totalement dérivant de polyaminoamides de formule générale :

$$\left[ CO\text{-}R_{19}\text{-}CO\text{-}Z \right] \qquad (X)$$

dans laquelle $R_{19}$ représente un radical divalent dérivé d'un acide dicarboxylique saturé, d'un acide aliphatique mono ou dicarboxylique à double liaison éthylénique, d'un ester d'un alcanol inférieur ayant 1 à 6 atomes de carbone de ces acides ou d'un radical dérivant de l'addition de l'un quelconque desdits acides avec une amine bis primaire ou bis secondaire, et Z désigne un radical d'une polyalkylène-polyamine bis-primaire, mono ou bis-secondaire et de préférence représente :

a) dans les proportions de 60 à 100 moles %, le radical

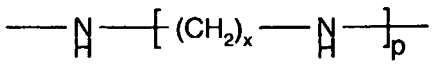

**(XI)**

où x=2 et p=2 ou 3, ou bien x=3 et p=2

ce radical dérivant de la diéthylène triamine, de la triéthylène tétraamine ou de la dipropylène triamine;

b) dans les proportions de 0 à 40 moles % le radical (XI) ci-dessus, dans lequel x=2 et p=1 et qui dérive de l'éthylènediamine, ou le radical dérivant de la pipérazine :

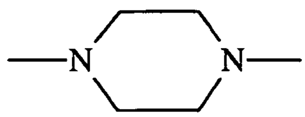

c) dans les proportions de 0 à 20 moles % le radical $-NH-(CH_2)_6-NH-$ dérivant de l'hexaméthylènediamine, ces polyaminoamines étant réticulées par addition d'un agent réticulant bifonctionnel choisi parmi les épihalohydrines, les diépoxydes, les dianhydrides, les dérivés bis insaturés, au moyen de 0,025 à 0,35 mole d'agent réticulant par groupement amine du polyaminoamide et alcoylés par action d'acide acrylique, d'acide chloracétique ou d'une alcane sultone ou de leurs sels.

Les acides carboxyliques saturés sont choisis de préférence parmi les acides ayant 6 à 10 atomes de carbone tels que l'acide adipique, triméthyl-2,2,4-adipique et triméthyl-2,4,4-adipique, téréphtalique, les acides à double liaison éthylénique comme par exemple les acides acrylique, méthacrylique, itaconique.

Les alcanes sultones utilisées dans l'alcoylation sont de préférence la propane ou la butane sultone, les sels des agents d'alcoylation sont de préférence les sels de sodium ou de potassium.

(4) Les polymères comportant des motifs zwittérioniques de formule :

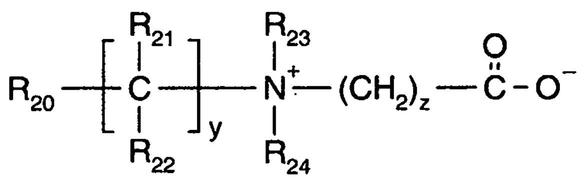

**(XII)**

dans laquelle $R_{20}$ désigne un groupement insaturé polymérisable tel qu'un groupement acrylate, méthacrylate, acrylamide ou méthacrylamide, y et z représentent un nombre entier de 1 à 3, $R_{21}$ et $R_{22}$ représentent un atome d'hydrogène, méthyle, éthyle ou propyle, $R_{23}$ et $R_{24}$ représentent un atome d'hydrogène ou un radical alkyle de telle façon que la somme des atomes de carbone dans $R_{23}$ et $R_{24}$ ne dépasse pas 10.

Les polymères comprenant de telles unités peuvent également comporter des motifs dérivés de monomères non zwittérioniques tels que l'acrylate ou le méthacrylate de diméthyl ou diéthylaminoéthyle ou des alkyle acrylates ou méthacrylates, des acrylamides ou méthacrylamides ou l'acétate de vinyle.
A titre d'exemple, on peut citer le copolymère de méthacrylate de butyle / méthacrylate de diméthylcarboxyméthylammonio-éthyle tel que le produit vendu sous la dénomination DIAFORMER Z301 par la société SANDOZ.

(5) les polymères dérivés du chitosane comportant des motifs monomères répondant aux formules (XIII), (XIV), (XV) suivantes :

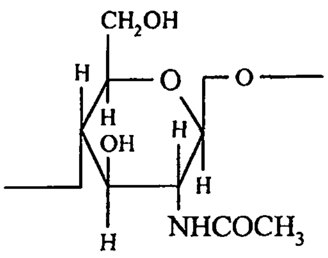

(XIII)

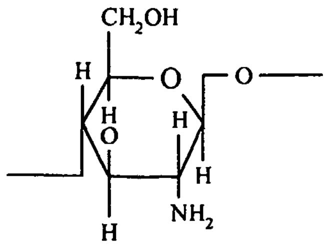

(XIV)

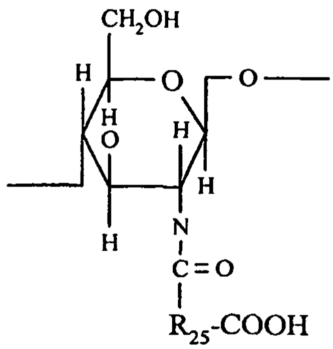

(XV)

le motif (XIII) étant présent dans des proportions comprises entre 0 et 30%, le motif (XIV) dans des proportions comprises entre 5 et 50% et le motif (XV) dans des proportions comprises entre 30 et 90%, étant entendu que dans ce motif (XV), $R_{25}$ représente un radical de formule :

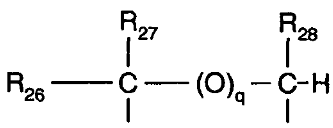

dans laquelle q= 0 ou 1,
si q=0, $R_{26}$, $R_{27}$ et $R_{28}$, identiques ou différents, représentent chacun un atome d'hydrogène, un reste méthyle, hydroxyle, acétoxy ou amino, un reste monoalcoylamine ou un reste dialcoylamine éventuellement interrompus par un ou plusieurs atomes d'azote et/ou éventuellement substitués par un ou plusieurs groupes amine, hydroxyle, carboxyle, alcoylthio, sulfonique, un reste alcoylthio dont le groupe alcoyle porte un reste amino, l'un au moins des radicaux $R_{26}$, $R_{27}$ et $R_{28}$ étant dans ce cas un atome d'hydrogène ;
ou si q=1, $R_{26}$, $R_{27}$ et $R_{28}$ représentent chacun un atome d'hydrogène, ainsi que les sels formés par ces composés avec des bases ou des acides.

(6) Les polymères dérivés de la N-carboxyalkylation du chitosane comme le N-carboxyméthyl chitosane ou le N-carboxybutyl chitosane vendu sous la dénomination "EVALSAN" par la société JAN DEKKER.

(7) Les polymères répondant à la formule générale (XI) tels que ceux décrits par exemple dans le brevet français 1 400 366 :

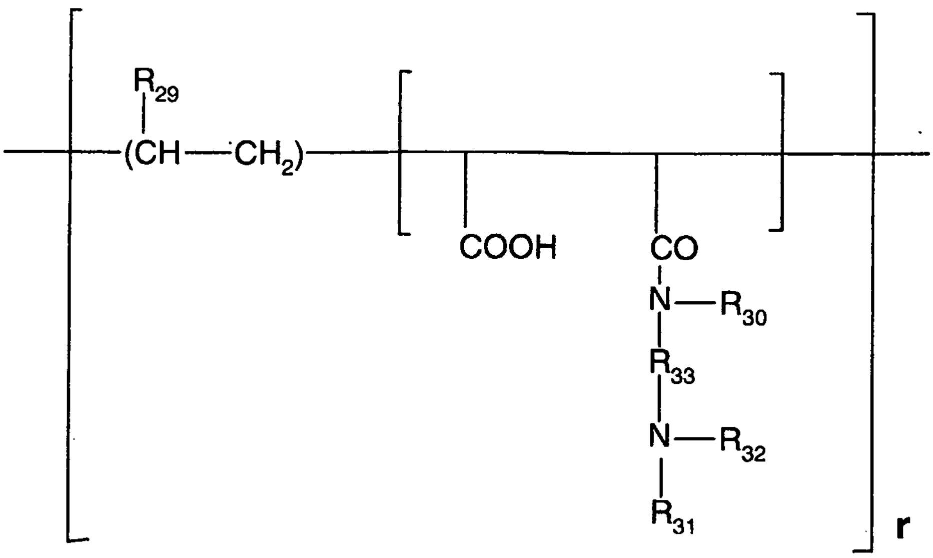

(XVI)

dans laquelle $R_{29}$ représente un atome d'hydrogène, un radical $CH_3O$, $CH_3CH_2O$, phényle, $R_{30}$ désigne l'hydrogène ou un radical alkyle inférieur tel que méthyle, éthyle, $R_{31}$ désigne l'hydrogène ou un radical alkyle inférieur tel que méthyle, éthyle, $R_{32}$ désigne un radical alkyle inférieur tel que méthyle, éthyle ou un radical répondant à la formule : $-R_{33}-N(R_{31})_2$, $R_{33}$ représentant un groupement $-CH_2-CH_2-$, $-CH_2-CH_2-CH_2-$ , $-CH_2-CH(CH_3)-$, $R_{31}$ ayant les significations mentionnées ci-dessus,
ainsi que les homologues supérieurs de ces radicaux et contenant jusqu'à 6 atomes de carbone,
r est tel que le poids moléculaire est compris entre 500 et 6000000 et de préférence entre 1000 et 1000000.

(8) Des polymères amphotères du type -D-X-D-X- choisis parmi:

a) les polymères obtenus par action de l'acide chloracétique ou le chloracétate de sodium sur les composés comportant au moins un motif de formule :

-D-X-D-X-D- (XVII)

où D désigne un radical

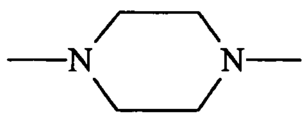

et X désigne le symbole E ou E', E ou E' identiques ou différents désignent un radical bivalent qui est un radical alkylène à chaîne droite ou ramifiée comportant jusqu'à 7 atomes de carbone dans la chaîne principale non substituée ou substituée par des groupements hydroxyle et pouvant comporter en outre des atomes d'oxygène, d'azote, de soufre, 1 à 3 cycles aromatiques et/ou hétérocycliques; les atomes d'oxygène, d'azote et de soufre étant présents sous forme de groupements éther, thioéther, sulfoxyde, sulfone, sulfonium, alkylamine, alkénylamine, des groupements hydroxyle, benzylamine, oxyde d'amine, ammonium quaternaire, amide, imide, alcool, ester et/ou uréthanne ;

b) les polymères de formule :

-D-X-D-X- (XVIII)

où D désigne un radical

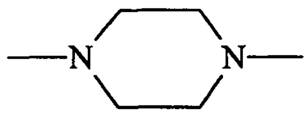

et X désigne le symbole E ou E' et au moins une fois E'; E ayant la signification indiquée ci-dessus et E' est un radical bivalent qui est un radical alkylène à chaîne droite ou ramifiée ayant jusqu'à 7 atomes de carbone dans la chaîne principale, substitué ou non par un ou plusieurs radicaux hydroxyle et comportant un ou plusieurs atomes d'azote, l'atome d'azote étant substitué par une chaîne alkyle interrompue éventuellement par un atome d'oxygène et comportant obligatoirement une ou plusieurs fonctions carboxyle ou une ou plusieurs fonctions hydroxyle et bétaïnisées par réaction avec l'acide chloracétique ou du chloracétate de soude.

(9) Les copolymères alkyl($C_1$-$C_5$)vinyléther / anhydride maléique modifié partiellement par semiamidification avec une N,N-dialkylaminoalkylamine telle que la N,N-diméthylaminopropylamine ou par semiestérification avec une N, N-dialcanolamine. Ces copolymères peuvent également comporter d'autres comonomères vinyliques tels que le vinylcaprolactame.

Les polymères amphotères particulièrement préférés selon l'invention sont ceux de la famille (1).

La composition peut encore comprendre une quantité efficace d'autres agents, par ailleurs antérieurement connus en coloration directe ou d'oxydation, tels que divers adjuvants usuels comme des séquestrants tel que l'EDTA et l'acide étidronique, des filtres UV, des cires, des silicones volatiles ou non, cycliques ou linéaires ou ramifiées, organomodifiées ou non et différentes de celles de la présente invention, des conservateurs, des céramides, des pseudocéramides, des huiles végétales, minérales ou de synthèse, les vitamines ou provitamines comme le panthénol, des opacifiants.

Ladite composition peut également comprendre des agents réducteurs ou antioxydants. Ceux-ci peuvent être choisis en particulier parmi le sulfite de sodium, l'acide thioglycolique, l'acide thiolactique, le bisulfite de sodium, l'acide déhydroascorbique, l'hydroquinone, la 2-méthyl-hydroquinone, la ter-butyl-hydroquinone et l'acide homogentisique, et ils sont alors généralement présents dans des quantités allant d'environ 0,05 à 3% en poids par rapport au poids total de la composition.

Bien entendu, l'homme de l'art veillera à choisir le ou les éventuels composés complémentaires mentionnés ci-avant, de manière telle que les propriétés avantageuses attachées intrinsèquement à la composition tinctoriale selon l'invention ne soient pas, ou substantiellement pas, altérées par la ou les adjonctions envisagées.

Dans la composition prête à l'emploi ou dans la composition oxydante, l'agent oxydant est choisi de préférence dans le groupe formé par le peroxyde d'urée, les bromates ou ferricyanures de métaux alcalins, les persels tels que les perborates et les persulfates. L'utilisation du peroxyde d'hydrogène est particulièrement préférée. Cet agent oxydant est avantageusement constitué par une solution d'eau oxygénée dont le titre peut varier, plus particulièrement, d'environ 1 à 40 volumes, et encore plus préférentiellement d'environ 5 à 40.

On peut également utiliser à titre d'agent oxydant une ou plusieurs enzymes d'oxydoréduction telles que les laccases, les peroxydases et les oxydoréductases à 2 électrons (telles que l'uricase), le cas échéant en présence de leur donneur ou cofacteur respectif.

Le pH de la composition prête à l'emploi et appliquée sur les fibres kératiniques [composition résultant du mélange de la composition tinctoriale et de la composition oxydante], est généralement compris entre les valeurs 4 et 11. Il est de préférence compris entre 6 et 10, et peut être ajusté à la valeur désirée au moyen d'agents acidifiants ou alcalinisants bien connus de l'état de la technique en teinture des fibres kératiniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxyalkylamines et les éthylènediamines oxyéthylénées et/ou oxypropylénées, les hydroxydes de sodium ou de potassium et les composés de formule (VI) suivante :

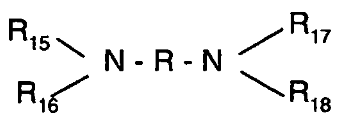

(VI)

dans laquelle R est un reste propylène éventuellement substitué par un groupement hydroxyle ou un radical alkyle en $C_1$-$C_4$ ; $R_{15}$, $R_{16}$, $R_{17}$ et $R_{18}$, identiques ou différents, représentent un atome d'hydrogène, un radical alkyle en $C_1$-$C_4$

ou hydroxyalkyle en $C_1$-$C_4$.

Les agents acidifiants sont classiquement, à titre d'exemple, des acides minéraux ou organiques comme l'acide chlorhydrique, l'acide orthophosphorique, des acides carboxyliques comme l'acide tartrique, l'acide citrique, l'acide lactique, ou des acides sulfoniques.

Le procédé de teinture selon l'invention consiste, de préférence, à appliquer la composition ou celle prête à l'emploi ( réalisée extemporanément au moment de l'emploi à partir des compositions colorantes et oxydantes décrites ci-avant), sur les fibres kératiniques sèches ou humides, et à la laisser agir pendant un temps de pause variant, de préférence, de 1 à 60 minutes environ, et plus préférentiellement de 10 à 45 minutes environ, à rincer les fibres, puis éventuellement à les laver au shampooing, puis à les rincer à nouveau, et à les sécher.

Des exemples concrets illustrant l'invention sont indiqués ci-après, sans pour autant présenter un caractère limitatif.

EXEMPLE 1:

On a préparé la composition de teinture directe suivante :
(exprimée en grammes de matière active)

| | |
|---|---|
| Colorant direct: Basic Blue 99........................................................................... | 0,1 |
| Polydiméthylsiloxane de formule (II) selon l'invention vendu sous la dénomination SLM 28020® par la société WACKER.................................. | 2 |
| Ethanol............................................................................................................ | 20 |
| Gomme de guar hydroxypropylée : Jaguar HP60® vendu par AQUALON...................................................................................................... | 1 |
| Alkyl (C8-C10) polyglucoside en solution aqueuse à 60% de matière active : Oramix CG110® vendu par SEPPIC............................................... | 8 |
| 2-amino-2-méthyl-1-propanol.....q.s.....pH........................................................... | 7,5 |
| Eau déminéralisée.....q.s.p.................................................................................. | 100 |

On a appliquée cette composition sur des mèches de cheveux gris naturels à 90% de blancs pendant 30 minutes. Les cheveux ont ensuite été rincés, lavés avec un shampooing standard, puis séchés.

On a obtenu une nuance bleue très résistante à plusieurs shampooings.
Par ailleurs, l'état cosmétique des fibres a été très satisfaisant.

EXEMPLE 2

On a préparé la teinture d'oxydation suivante :
(exprimée en grammes de matière active)

| | |
|---|---|
| Paraphénylènediamine......................................................................................... | 0,108 |
| 2-méthyl-5-amino-phénol..................................................................................... | 0,123 |
| Polydiméthylsiloxane de formule (I) selon l'invention proposé sous la dénomination BELSIL ADM 652® par la société WACKER......................... | 2 |
| Ethano.................................................................................................................. | 20 |
| Alkyl (C8-C10) polyglucoside en solution aqueuse à 60% de matière active : Oramix CG110® vendu par SEPPIC.................................................. | 3,6 |
| Alcool benzylique.................................................................................................. | 2 |
| Polyéthylèneglycol à 8 moles d'oxyde d'éthylène........................................... | 3 |
| Métabisulfite de sodium en solution aqueuse à 35% de matière active | 0,227 |
| Sel pentasodique de l'acide diéthylène triamine pentacétique................... | 0,48 |
| Ammoniaque à 20% en NH3............................................................................. | 6,8 |

(suite)

| | |
|---|---|
| Eau déminéralisée.................. q.s.p..................................................................... | 100 |

On a mélangé cette composition, poids pour poids, avec de l'eau oxygénée à 20 volumes. Le pH final du mélange a été de 9,5.
Puis on appliqué le mélange sur des cheveux gris à 90% de blancs et laissé poser pendant 30 minutes.
Les cheveux ont ensuite été lavés avec un shampooing standard puis rincés à l'eau et séchés.
Ils ont été teints dans une nuance rouge-violacé, résistante à plusieurs shampooings, et les fibres ont présenté un état cosmétique très satisfaisant.

## Revendications

1. Composition de teinture pour fibres kératiniques humaines, comprenant, dans un milieu cosmétiquement acceptable, au moins un colorant direct ou au moins un colorant d'oxydation, qui est **caractérisée par le fait qu'**elle contient en outre au moins une silicone aminée de formules (I) ou (II) suivantes :

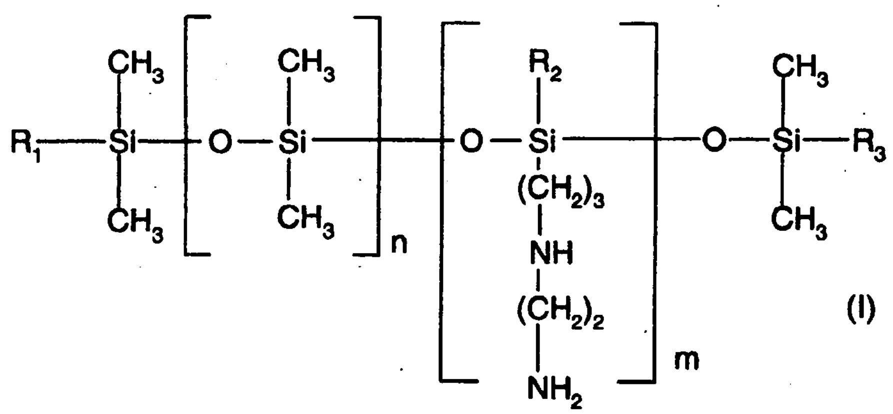

formule (I) dans laquelle :
m et n sont des nombres tels que la somme (n + m) varie de 1 à 1 000,
n désignant un nombre de 0 à 999 et m désignant un nombre de 1 à 1 000;
$R_1$, $R_2$, $R_3$, identiques ou différents, représentent un radical hydroxy ou alcoxy en $C_1$-$C_4$ ; l'un au moins des radicaux $R_1$ à $R_3$ désignant un radical alcoxy ;

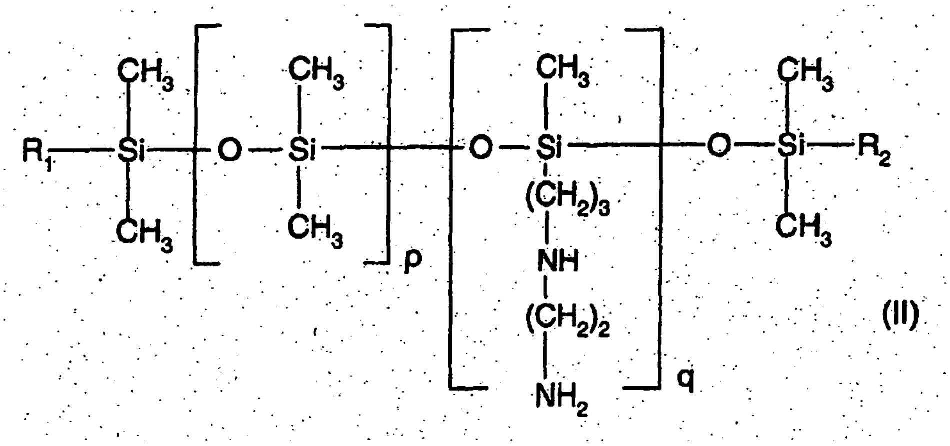

formule (II) dans laquelle :

p et q sont des nombres tels que la somme (p + q) varie de 1 à 1 000,
p désignant un nombre de 0 à 999
et q désignant un nombre de 1 à 1 000 ;
$R_1$, $R_2$, différents, représentent un radical hydroxy ou alcoxy en $C_1$-$C_4$, l'un au moins des radicaux $R_1$ à $R_2$ désignant un radical alcoxy.

2. Composition selon la revendication 1, **caractérisée par le fait que** le radical alcoxy $C_1$-$C_4$ désigne le radical méthoxy.

3. Composition selon l'une quelconque des revendications précédentes, **caractérisée par le fait que** pour les silicones aminées de formule (I), le rapport molaire Hydroxy/Alcoxy est compris entre 0,2:1 et 0,4:1.

4. Composition selon l'une quelconque des revendications 1 à 2, **caractérisée par le fait que** pour les silicones aminées de formule (II), le rapport molaire Hydroxy/Alcoxy est compris entre 1:0,8 et 1:1,1.

5. Composition selon l'une quelconque des revendications 1 à 3, **caractérisée par le fait que** la silicone aminée de formule (I) présente une masse moléculaire moyenne en poids allant de 2000 à 1000000.

6. Composition selon l'une quelconque des revendications 1 à 2 et 4, **caractérisée par le fait que** la silicone aminée de formule (II) présente une masse moléculaire moyenne en poids allant de 2000 à 200.000.

7. Composition selon l'une quelconque des revendications précédentes, **caractérisée par le fait que** la silicone aminée de formule (I) ou (II) est sous forme d'émulsion huile-dans-eau comprenant des agents tensioactifs.

8. Composition selon la revendication 7, **caractérisée par le fait que** l'émulsion comprend au moins un agent tensioactif cationique et/ou non ionique.

9. Composition selon l'une quelconque des revendications 7 ou 8, **caractérisée par le fait que** les particules de Silicone dans l'émulsion ont une taille allant de 3 nm à 500 nanomètres.

10. Composition selon l'une quelconque des revendications précédentes, **caractérisée par le fait que** la silicone aminée de formule (I) ou (II) est présente dans la composition de teinture en une quantité allant de 0,01 à 20% en poids du poids total de la composition.

11. Composition selon la revendication 10, **caractérisée par le fait qu'**elle est présente en une quantité allant de 0,1 à 15% en poids du poids total de la composition.

12. Composition selon la revendication 11, **caractérisée par le fait qu'**elle est présente en une quantité allant de 0,5 à 10 % en poids du poids total de la composition.

13. Composition selon l'une quelconque des revendications précédentes, **caractérisée par le fait que** le colorant d'oxydation est choisi parmi les bases d'oxydation et/ou les coupleurs.

14. Composition selon la revendication 13, **caractérisée par le fait qu'**elle comprend au moins une base d'oxydation.

15. Composition selon la revendication 14, **caractérisée par le fait que** les bases d'oxydation sont choisies parmi les ortho- ou para- phénylènediamines, les bases doubles, les ortho- ou para- aminophénols, et les bases hétérocycliques, ainsi que les sels d'addition de ces composés avec un acide.

16. Composition selon la revendication 15, **caractérisée par le fait que** les paraphénylènediamines sont choisies parmi les composés de structure (III) suivante :

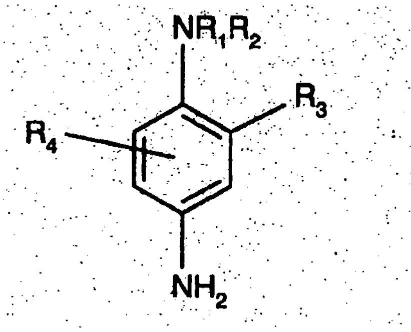

(III)

dans laquelle :

$R_1$ représente un atome d'hydrogène, un radical alkyle en $C_1$-$C_4$, monohydroxyalkyle en $C_1$-$C_4$, polyhydroxyalkyle en $C_2$-$C_4$, alcoxy($C_1$-$C_4$)alkyle($C_1$-$C_4$), alkyle en $C_1$-$C_4$ substitué par un groupement azoté, phényle ou 4'-aminophényle ;

$R_2$ représente un atome d'hydrogène, un radical alkyle en $C_1$-$C_4$, monohydroxyalkyle en $C_1$-$C_4$ ou polyhydroxyalkyle en $C_2$-$C_4$, alcoxy($C_1$-$C_4$)alkyle($C_1$-$C_4$) ou alkyle en $C_1$-$C_4$ substitué par un groupement azoté ;

$R_1$ et $R_2$ peuvent également former avec l'atome d'azote qui les porte un hétérocycle azoté à 5 ou 6 chaînons éventuellement substitué par un ou plusieurs groupements alkyle, hydroxy ou uréido;

$R_3$ représente un atome d'hydrogène, un atome d'halogène tel qu'un atome de chlore, un radical alkyle en $C_1$-$C_4$, sulfo, carboxy, monohydroxyalkyle en $C_1$-$C_4$ ou hydroxyalcoxy en $C_1$-$C_4$, acétylaminoalcoxy en $C_1$-$C_4$, mésylaminoalcoxy en $C_1$-$C_4$ ou carbamoylaminoalcoxy en $C_1$-$C_4$,

$R_4$ représente un atome d'hydrogène, d'halogène ou un radical alkyle en $C_1$-$C_4$

**17.** Composition selon la revendication 15, **caractérisée par le fait que** les bases doubles sont choisies parmi les composés de structure (IV) suivante :

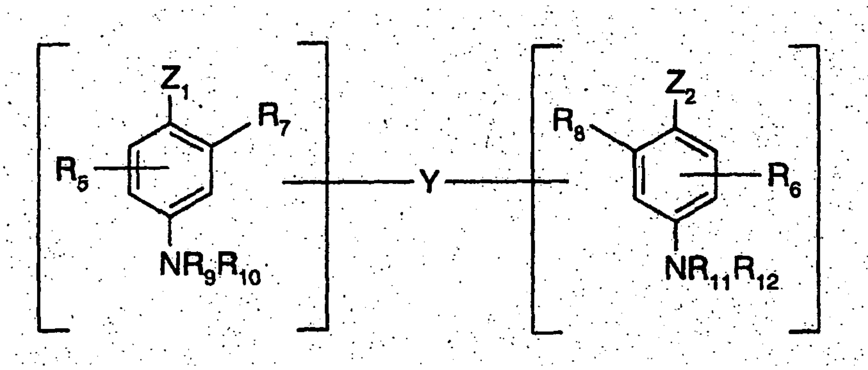

(IV)

dans laquelle :

- $Z_1$ et $Z_2$, identiques ou différents, représentent un radical hydroxyle pu -$NH_2$ pouvant être substitué par un radical alkyle en $C_1$-$C_4$ ou par un bras de liaison Y ;
- le bras de liaison Y représente une chaîne alkylène comportant de 1 à 14 atomes de carbone, linéaire ou ramifiée pouvant être interrompue ou terminée par un ou plusieurs groupements azotés et/ou par un ou plusieurs hétéroatomes tels que des atomes d'oxygène, de soufre ou d'azote, et éventuellement substituée par un ou plusieurs radicaux hydroxyle ou alcoxy en $C_1$-$C_6$ ;
- $R_5$ et $R_6$ représentent un atome d'hydrogène ou d'halogène, un radical alkyle en $C_1$-$C_4$, monohydroxyalkyle en $C_1$-$C_4$, polyhydroxyalkyle en $C_2$-$C_4$, aminoalkyle en $C_1$-$C_4$ ou un bras de liaison Y ;
- $R_7$, $R_8$, $R_9$, $R_{10}$, $R_{11}$ et $R_{12}$, identiques ou différents, représentent un atome d'hydrogène, un bras de liaison Y ou un radical alkyle en $C_1$-$C_4$ ;

étant entendu que les composés de formule (IV) ne comportent qu'un seul bras de liaison Y par molécule.

**18.** Composition selon la revendication 15, **caractérisée par le fait que** les paraaminophénols sont choisis parmi les

composés de structure (V) suivante :

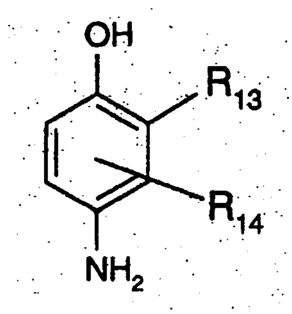

(V)

dans laquelle :

$R_{13}$ représente un atome d'hydrogène,un atome d'halogène tel que le fluor, un radical alkyle en $C_1$-$C_4$, monohydroxyalkyle en $C_1$-$C_4$, alcoxy($C_1$-$C_4$)alkyle($C_1$-$C_4$) ou aminoalkyle en $C_1$-$C_4$, ou hydroxyalkyl($C_1$-$C_4$)aminoalkyle en $C_1$-$C_4$.

$R_{14}$ représente un atome d'hydrogène ou un atome d'halogène tel que le fluor, un radical alkyle en $C_1$-$C_4$, monohydroxyalkyle en $C_1$-$C_4$, polyhydroxyalkyle en $C_2$-$C_4$, aminoalkyle en $C_1$-$C_4$, cyanoalkyle en $C_1$-$C_4$ ou alcoxy($C_1$-$C_4$)alkyle($C_1$-$C_4$).

**19.** Composition selon la revendication 15, **caractérisée par le fait que** les bases hétérocycliques sont choisies parmi les dérivés pyridiniques, les dérivés pyrimidiniques dont les pyrazolopyrimidines, les dérivés pyrazoliques.

**20.** Composition selon l'une quelconque des revendications 13 à 19, **caractérisée par le fait que** les bases d'oxydation sont présentes dans des concentrations allant de 0,0005 à 12% en poids par rapport au poids total de la composition.

**21.** Composition selon la revendication 13, **caractérisée par le fait que** les coupleurs sont choisis parmi les métaphénylènediamines, les méta-aminophénols, les métadiphénols, les coupleurs hétérocycliques, et les sels d'addition de ces composés avec un acide.

**22.** Composition selon l'une quelconque des revendications 13 or 21, **caractérisée par le fait que** les coupleurs sont présents dans des concentrations allant de 0,0001 à 10% en poids par rapport au poids total de la composition.

**23.** Composition selon l'une quelconque des revendications 13 à 22, **caractérisée par le fait que** les sels d'addition avec un acide des colorants d'oxydation sont choisis parmi les chlorhydrates, les bromhydrates, les sulfates, les tartrates, les lactates et les acétates.

**24.** Composition selon l'une quelconque des revendications précédentes, **caractérisée par le fait que** le colorant direct est choisi parmi les colorants directs nitrés benzéniques neutres, acides ou cationiques, les colorants directs azoïques neutres, acides ou cationiques, les colorants directs quinoniques, les colorants directs aziniques, les colorants directs triarylméthaniques, les colorants directs indoaminiques et les colorants directs naturels.

**25.** Composition selon la revendication précédente **caractérisée par le fait que** le colorant direct est choisi parmi les colorants directs anthraquinoniques, neutres, acides ou cationiques.

**26.** Composition selon l'une quelconque des revendications précédentes, **caractérisée par le fait que** le ou les colorants directs sont présents dans des concentrations allant de 0,001 à 20% en poids par rapport au poids total de la composition.

**27.** Composition selon l'une quelconque des revendications précédentes, **caractérisée par le fait qu'**elle contient en outre au moins un agent réducteur, dans des quantités allant de 0,05 à 3% en poids par rapport au poids total de la composition.

**28.** Composition selon l'une quelconque des revendications précédentes, **caractérisée par le fait qu'**elle contient en outre un agent oxydant et qu'elle est prête à l'emploi.

**29.** Composition selon la revendication 28 **caractérisée par le fait que** l'agent oxydant est choisi parmi le peroxyde d'hydrogène, le peroxyde d'urée, les bromates ou ferricyanures de métaux alcalins, les persels, les enzymes d'oxydoréduction avec éventuellement leur donneur ou cofacteur respectif.

**30.** Composition selon la revendication 29, **caractérisée par le fait que** l'agent oxydant est le peroxyde d'hydrogène.

**31.** Composition selon la revendication 30 **caractérisée par le fait que** l'agent oxydant est une solution d'eau oxygénée dont le titre varie de 1 à 40 volumes.

**32.** Composition selon l'une quelconque des revendications précédentes, **caractérisée par le fait qu'**elle possède un pH allant de 4 à 11.

**33.** Procédé de teinture des fibres kératiniques humaines, **caractérisé par le fait qu'**il consiste à appliquer sur les fibres une composition colorante comprenant, dans un milieu cosmétiquement acceptable, au moins un colorant direct et en outre au moins une silicone aminée de formule (I) ou (II) telle que définie à l'une quelconque des revendications 1 à 12.

**34.** Procédé de teinture des fibres kératiniques humaines, **caractérisé par le fait qu'**il consiste à appliquer sur les fibres au moins une composition colorante comprenant, dans un milieu cosmétiquement acceptable, au moins un colorant direct ou au moins un colorant d'oxydation, et en outre au moins une silicone aminée de formule (I) ou (II) telle que définie à l'une quelconque des revendications 1 à 12, , la couleur étant révélée à pH alcalin, neutre ou acide, à l'aide d'une composition comprenant au moins un agent oxydant qui est mélangée juste au moment de l'emploi à la composition colorante ou qui est appliquée séquentiellement sans rinçage intermédiaire.

**35.** Procédé selon la revendication 34, **caractérisé par le fait qu'**il consiste à appliquer sur les fibres kératiniques sèches ou humides, la composition colorante ou celle prête à l'emploi, réalisée extemporanément au moment de l'emploi à partir des compositions colorante et oxydante, à la laisser agir pendant un temps de pause variant de 1 à 60 minutes environ, à rincer les fibres, puis éventuellement à les laver au shampooing, puis à les rincer à nouveau, et à les sécher.

**36.** Dispositif à 2 compartiments ou « Kit » pour la teinture des fibres kératiniques humaines, **caractérisé par le fait qu'**il comprend un compartiment renfermant une composition comprenant, dans un milieu cosmétiquement acceptable, au moins un colorant direct ou au moins un colorant d'oxydation, et au moins une silicone aminée de formule (I) ou (II) telle que définie à l'une quelconque des revendications 1 à 12, et un autre compartiment renfermant une composition comprenant, dans un milieu cosmétiquement acceptable, un agent oxydant.

**37.** Dispositif à 3 compartiments ou « Kit » pour la teinture des fibres kératiniques humaines, **caractérisé par le fait qu'**il comprend un premier compartiment renfermant une composition comprenant, dans un milieu cosmétiquement acceptable, au moins un colorant direct ou au moins un colorant d'oxydation, un deuxième compartiment renfermant une composition comprenant, dans un milieu cosmétiquement acceptable, au moins un agent oxydant, et un troisième compartiment renfermant une composition comprenant, dans un milieu cosmétiquement acceptable, au moins une silicone aminée de formule (I) ou (II) telle que définie à l'une quelconque des revendications 1 à 12.

## Claims

**1.** Composition for dyeing human keratin fibres, comprising in a cosmetically acceptable medium at least one direct dye or at least one oxidation dye, **characterized in that** it further comprises at least one amino silicone of formula (I) or (II) below:

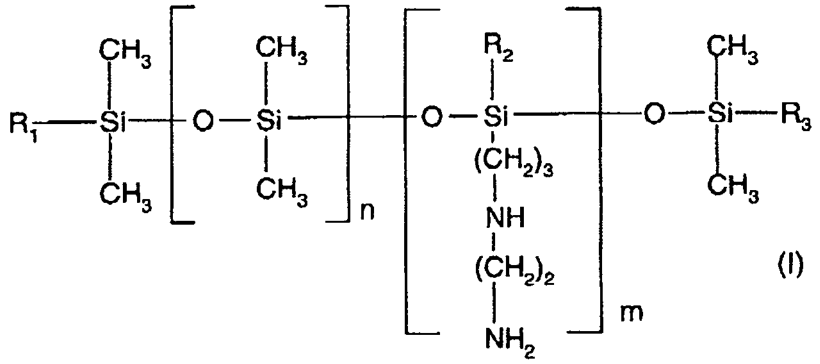

(I)

in which formula (I):

m and n are numbers such that the sum (n + m) varies from 1 to 1000, n denoting a number from 0 to 999 and m denoting a number from 1 to 1000; $R_1$, $R_2$ and $R_3$, which are identical or different, represent a hydroxyl or $C_1$-$C_4$ alkoxy radical, at least one of the radicals $R_1$ to $R_3$ denoting an alkoxy radical;

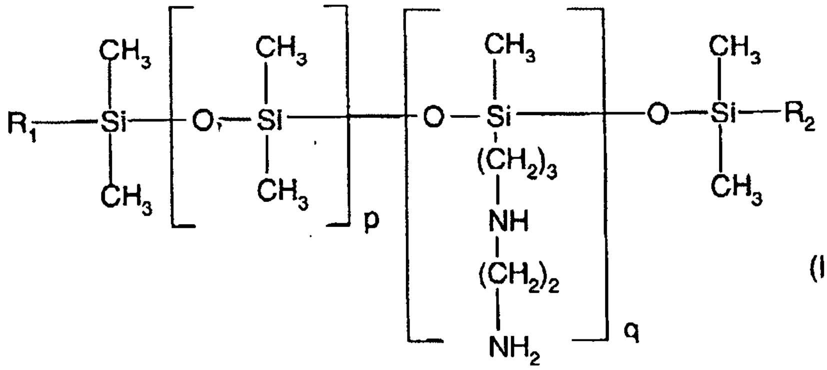

(II)

in which formula (II):

p and q are numbers such that the sum (p + q) varies from 1 to 1000, p denoting a number from 0 to 999 and q denoting a number from 1 to 1000; $R_1$ and $R_2$, which are different, represent a hydroxyl or $C_1$-$C_4$ alkoxy radical, at least one of the radicals $R_1$ and $R_2$ denoting an alkoxy radical.

2. Composition according to Claim 1, **characterized in that** the $C_1$-$C_4$ alkoxy radical denotes the methoxy radical.

3. Composition according to either of the preceding claims, **characterized in that** for the amino silicones of formula (I) the hydroxy/alkoxy molar ratio is between 0.2:1 and 0.4:1.

4. Composition according to either of Claims 1 and 2, **characterized in that** for the amino silicones of formula (II) the hydroxy/alkoxy molar ratio is between 1:0.8 and 1:1.1.

5. Composition according to any one of Claims 1 to 3, **characterized in that** the amino silicone of formula (I) has a weight-average molecular mass ranging from 2000 to 1 000 000.

6. Composition according to any one of Claims 1 to 2 and 4, **characterized in that** the amino silicone of formula (II) has a weight-average molecular mass ranging from 2000 to 200 000.

**7.** Composition according to any one of the preceding claims, **characterized in that** the amino silicone of formula (I) or (II) is in the form of an oil-in-water emulsion comprising surfactants.

**8.** Composition according to Claim 7, **characterized in that** the emulsion comprises at least one cationic and/or nonionic surfactant.

**9.** Composition according to either of Claims 7 and 8, **characterized in that** the particles of silicone in the emulsion have a size ranging from 3 nm to 500 nanometres.

**10.** Composition according to any one of the preceding claims, **characterized in that** the amino silicone of formula (I) or (II) is present in the dyeing composition in an amount ranging from 0.01 to 20% by weight of the total weight of the composition.

**11.** Composition according to Claim 10, **characterized in that** it is present in an amount ranging from 0.1 to 15% by weight of the total weight of the composition.

**12.** Composition according to Claim 11, **characterized in that** it is present in an amount ranging from 0.5 to 10% by weight of the total weight of the composition.

**13.** Composition according to any one of the preceding claims, **characterized in that** the oxidation dye is selected from oxidation bases and/or couplers.

**14.** Composition according to Claim 13, **characterized in that** it comprises at least one oxidation base.

**15.** Composition according to Claim 14, **characterized in that** the oxidation bases are selected from ortho- and para-phenylenediamines, double bases, ortho- and para-aminophenols, heterocyclic bases, and the addition salts of these compounds with an acid.

**16.** Composition according to Claim 15, **characterized in that** the para-phenylenediamines are selected from the compounds of structure (III) below:

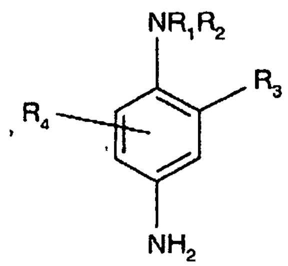

**(III)**

in which:

$R_1$ represents a hydrogen atom or a $C_1$-$C_4$ alkyl, $C_1$-$C_4$ monohydroxyalkyl, $C_2$-$C_4$ polyhydroxyalkyl, ($C_1$-$C_4$) alkoxy($C_1$-$C_4$)alkyl, $C_1$-$C_4$ alkyl substituted by a nitrogenous group, phenyl or 4-aminophenyl radical;
$R_2$ represents a hydrogen atom or a $C_1$-$C_4$ alkyl, $C_1$-$C_4$ monohydroxyalkyl or $C_2$-$C_4$ polyhydroxyalkyl radical or ($C_1$-$C_4$)alkoxy($C_1$-$C_4$)alkyl or $C_1$-$C_4$ alkyl substituted by a nitrogenous group;
$R_1$ and $R_2$ may also form, with the nitrogen atom which bears them, a 5- or 6-membered nitrogenous heterocycle optionally substituted by one or more alkyl, hydroxyl or ureido groups;
$R_3$ represents a hydrogen atom, a halogen atom such as a chlorine atom or a $C_1$-$C_4$ alkyl, sulpho, carboxyl, $C_1$-$C_4$ monohydroxyalkyl or $C_1$-$C_4$ hydroxyalkoxy, $C_1$-$C_4$ acetylaminoalkoxy, $C_1$-$C_4$ mesylaminoalkoxy or $C_1$-$C_4$ carbamoylaminoalkoxy radical, and
$R_4$ represents a hydrogen or halogen atom or a $C_1$-$C_4$ alkyl radical.

**17.** Composition according to Claim 15, **characterized in that** the double bases are selected from the compounds of structure (IV) below:

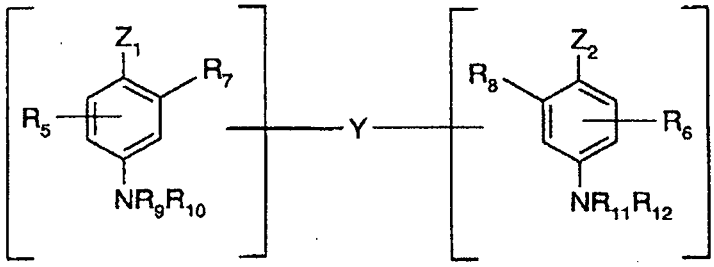 (IV)

in which:

- $Z_1$ and $Z_2$, which are identical or different, represent a hydroxyl or $-NH_2$ radical which may be substituted by a $C_1$-$C_4$ alkyl radical or by a linking arm Y;
- the linking arm Y represents an alkylene chain containing from 1 to 14 carbon atoms which is linear or branched and may be interrupted or terminated by one or more nitrogenous groups and/or by one or more heteroatoms such as oxygen, sulphur or nitrogen atoms and which is optionally substituted by one or more hydroxyl or $C_1$-$C_6$ alkoxy radicals;
- $R_5$ and $R_6$ represent a hydrogen or halogen atom, a $C_1$-$C_4$ alkyl, $C_1$-$C_4$ monohydroxyalkyl, $C_2$-$C_4$ polyhydroxyalkyl or $C_1$-$C_4$ aminoalkyl radical or a linking arm Y;
- $R_7$, $R_8$, $R_9$, $R_{10}$, $R_{11}$ and $R_{12}$, which are identical or different, represent a hydrogen atom, a linking arm Y or a $C_1$-$C_4$ alkyl radical;

subject to the proviso that the compounds of formula (IV) contain only one linking arm Y per molecule.

18. Composition according to Claim 15, **characterized in that** the para-aminophenols are selected from the compounds of structure (V) below:

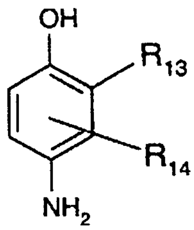 (V)

in which:

$R_{13}$ represents a hydrogen atom, a halogen atom such as fluorine or a $C_1$-$C_4$ alkyl, $C_1$-$C_4$ monohydroxyalkyl, $(C_1$-$C_4)$ alkoxy$(C_1$-$C_4)$ alkyl, $C_1$-$C_4$ aminoalkyl or $C_1$-$C_4$ hydroxy$(C_1$-$C_4)$alkylaminoalkyl radical and
$R_{14}$ represents a hydrogen atom, a halogen atom such as fluorine or a $C_1$-$C_4$ alkyl, $C_1$-$C_4$ monohydroxyalkyl, $C_2$-$C_4$ polyhydroxyalkyl, $C_1$-$C_4$ aminoalkyl, $C_1$-$C_4$ cyanoalkyl or $(C_1$-$C_4)$alkoxy$(C_1$-$C_4)$alkyl radical.

19. Composition according to Claim 15, **characterized in that** the heterocyclic bases are selected from pyridine derivatives, pyrimidine derivatives including pyrazolopyrimidines, and pyrazole derivatives.

20. Composition according to any one of Claims 13 to 19, **characterized in that** the oxidation bases are present in concentrations ranging from 0.0005 to 12% by weight relative to the total weight of the composition.

21. Composition according to Claim 13, **characterized in that** the couplers are selected from meta-phenylenediamines, meta-aminophenols, metadiphenols, heterocyclic couplers, and the addition salts of these compounds with an acid.

22. Composition according to either of Claims 13 and 21, **characterized in that** the couplers are present in concentrations ranging from 0.0001 to 10% by weight relative to the total weight of the composition.

**23.** Composition according to any one of Claims 13 to 22, **characterized in that** the addition salts with an acid of the oxidation dyes are selected from hydrochlorides, hydrobromides, sulphates, tartrates, lactates and acetates.

**24.** Composition according to any one of the preceding claims, **characterized in that** the direct dye is selected from neutral, acidic or cationic nitro benzene direct dyes, neutral, acidic or cationic azo direct dyes, quinone direct dyes, azine direct dyes, triarylmethane direct dyes, indoamine direct dyes and natural direct dyes.

**25.** Composition according to the preceding claim, **characterized in that** the direct dye is selected from neutral, acidic or cationic anthraquinone direct dyes.

**26.** Composition according to any one of the preceding claims, **characterized in that** the direct dye or dyes is or are present in concentrations ranging from 0.001 to 20% by weight relative to the total weight of the composition.

**27.** Composition according to any one of the preceding claims, **characterized in that** it further comprises at least one reducing agent in amounts ranging from 0.05 to 3% by weight relative to the total weight of the composition.

**28.** Composition according to any one of the preceding claims, **characterized in that** it further comprises an oxidizing agent and that it is ready to use.

**29.** Composition according to Claim 28, **characterized in that** the oxidizing agent is selected from hydrogen peroxide, urea peroxide, alkali metal bromates or ferricyanides, persalts, and redox enzymes present where appropriate with their respective donor or cofactor.

**30.** Composition according to Claim 29, **characterized in that** the oxidizing agent is hydrogen peroxide.

**31.** Composition according to Claim 30, **characterized in that** the oxidizing agent is a hydrogen peroxide solution whose titre varies from 1 to 40 volumes.

**32.** Composition according to any one of the preceding claims, **characterized in that** it has a pH ranging from 4 to 11.

**33.** Method of dyeing human keratin fibres, **characterized in that** it consists in applying to the fibres a colouring composition comprising in a cosmetically acceptable medium at least one direct dye and, furthermore, at least one amino silicone of formula (I) or (II) as defined in any one of Claims 1 to 12.

**34.** Method of dyeing human keratin fibres, **characterized in that** it consists in applying to the fibres at least one colouring composition comprising in a cosmetically acceptable medium at least one direct dye or at least one oxidation dye and, furthermore, at least one amino silicone of formula (I) or (II) as defined in any one of Claims 1 to 12, the colour being developed at alkaline, neutral or acidic pH by means of a composition comprising at least one oxidizing agent, which is mixed at the time of use with the colouring composition or which is applied sequentially without rinsing in between.

**35.** Method according to Claim 34, **characterized in that** it consists in applying to the dry or wet keratin fibres the colouring composition or the ready-to-use composition, produced extemporaneously at the time of use from the colouring and oxidizing compositions, and in leaving the said composition to act for a waiting time varying from 1 to 60 minutes approximately, in rinsing the fibres, then optionally washing them with shampoo, rinsing them again and drying them.

**36.** 2-Compartment device or kit for dyeing human keratin fibres, **characterized in that** it comprises one compartment containing a composition comprising in a cosmetically acceptable medium at least one direct dye or at least one oxidation dye and at least one amino silicone of formula (I) or (II) as defined in any one of Claims 1 to 12 and another compartment containing a composition comprising in a cosmetically acceptable medium an oxidizing agent.

**37.** 3-Compartment device or kit for dyeing human keratin fibres, **characterized in that** it comprises a first compartment containing a composition comprising in a cosmetically acceptable medium at least one direct dye or at least one oxidation dye, a second compartment containing a composition comprising in a cosmetically acceptable medium at least one oxidizing agent, and a third compartment containing a composition comprising in a cosmetically acceptable medium at least one amino silicone of formula (I) or (II) as defined in any one of Claims 1 to 12.

**Patentansprüche**

1. Zusammensetzung zum Färben menschlicher Keratinfasern, die in einem kosmetisch akzeptablen Medium mindestens einen Direktfarbstoff oder mindestens einen Oxidationsfarbstoff enthält, **dadurch gekennzeichnet, dass** sie ferner mindestens ein aminiertes Silicon der folgenden Formeln (I) oder (II) enthält:

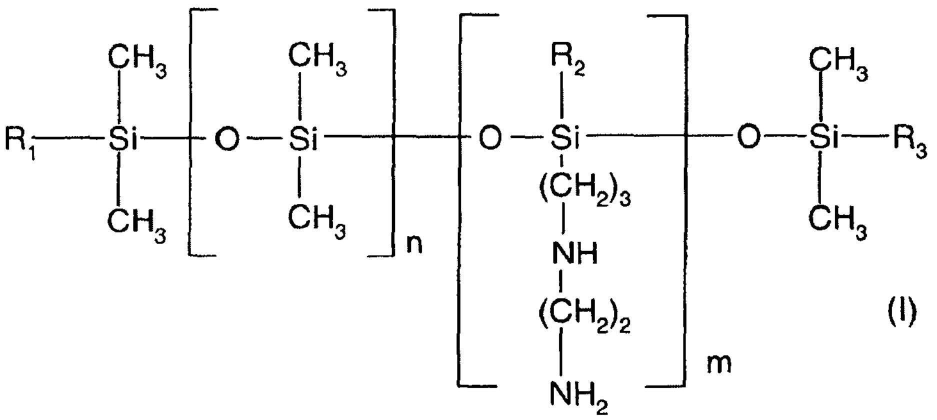

(I)

in der Formel (I):

m und n bedeuten Zahlen, die so gewählt sind, dass die Summe (n + m) im Bereich von 1 bis 1000 liegt,
n ist eine Zahl im Bereich von 0 bis 999 und m ist eine Zahl im Bereich von 1 bis 1000,
$R_1$, $R_2$ und $R_3$, die gleich oder verschieden sind, bedeuten eine Hydroxygruppe oder eine $C_{1-4}$-Alkoxygruppe, wobei mindestens eine der Gruppen $R_1$ bis $R_3$ eine Alkoxygruppe bedeutet;

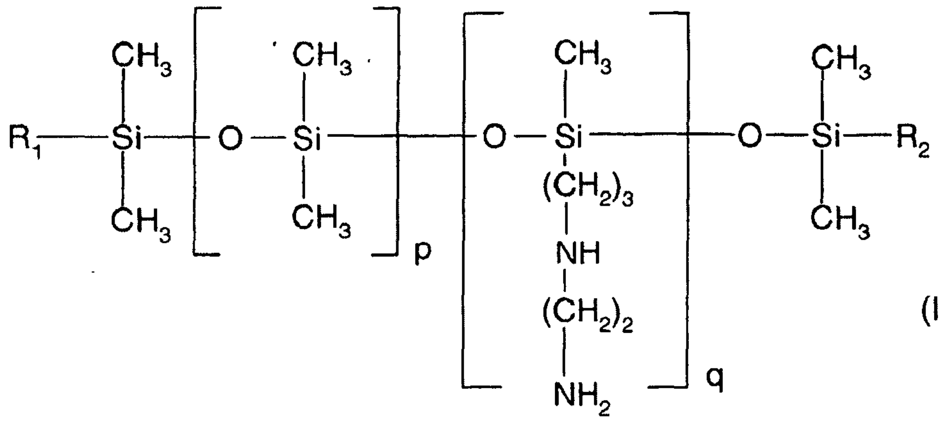

(II)

in der Formel (II):

p und q bedeuten Zahlen, die so gewählt sind, dass die Summe (p + q) im Bereich von 1 bis 1000 liegt,
p ist eine Zahl im Bereich von 0 bis 999 und q ist eine Zahl im Bereich von 1 bis 1000,
$R_1$ und $R_2$, die verschieden sind, bedeuten eine Hydroxygruppe oder eine $C_{1-4}$-Alkoxygruppe, wobei mindestens eine der Gruppen $R_1$ bis $R_2$ eine Alkoxygruppe bedeutet.

2. Zusammensetzung nach Anspruch 1, **dadurch gekennzeichnet, dass** die $C_{1-4}$-Alkoxygruppe die Methoxygruppe bedeutet.

3. Zusammensetzung nach einem der vorhergehenden Ansprüche, **dadurch gekennzeichnet, dass** für die aminierten Silicone der Formel (I) das Molverhältnis Hydroxy/Alkoxy im Bereich von 0,2:1 bis 0,4:1 liegt.

4. Zusammensetzung nach einem der Ansprüche 1 bis 2, **dadurch gekennzeichnet, dass** für die aminierten Silicone der Formel (II) das Molverhältnis Hydroxy/Alkoxy im Bereich von 1:0,8 bis 1:1,1 liegt.

5. Zusammensetzung nach einem der Ansprüche 1 bis 3, **dadurch gekennzeichnet, dass** das aminierte Silicon der Formel (I) eine gewichtmittlere Molmasse von 2.000 bis 1.000.000 aufweist.

6. Zusammensetzung nach einem der Ansprüche 1 bis 2 und 4, **dadurch gekennzeichnet, dass** das aminierte Silicon der Formel (II) eine gewichtmittlere Molmasse von 2.000 bis 200.000 aufweist.

7. Zusammensetzung nach einem der vorhergehenden Ansprüche, **dadurch gekennzeichnet, dass** das aminierte Silicon der Formel (I) oder (II) in Form einer Öl-in-Wasser-Emulsion vorliegt, die grenzflächenaktive Stoffe enthält.

8. Zusammensetzung nach Anspruch 7, **dadurch gekennzeichnet, dass** die Emulsion mindestens einen kationischen und/oder nichtionischen grenzflächenaktiven Stoff enthält.

9. Zusammensetzung nach einem der Ansprüche 7 oder 8, **dadurch gekennzeichnet, dass** die Siliconpartikel in der Emulsion eine Größe im Bereich von 3 bis 500 nm aufweisen.

10. Zusammensetzung nach einem der vorhergehenden Ansprüche, **dadurch gekennzeichnet, dass** das aminierte Silicon der Formel (I) oder (II) in der Farbmittelzusammensetzung in einem Mengenanteil von 0,01 bis 20 Gew.-% des Gesamtgewichts der Zusammensetzung enthalten ist.

11. Zusammensetzung nach Anspruch 10, **dadurch gekennzeichnet, dass** es in einem Mengenanteil von 0,1 bis 15 Gew.-% des Gesamtgewichts der Zusammensetzung enthalten ist.

12. Zusammensetzung nach Anspruch 11, **dadurch gekennzeichnet, dass** es in einem Mengenanteil von 0,5 bis 10 Gew.-% des Gesamtgewichts der Zusammensetzung enthalten ist.

13. Zusammensetzung nach einem der vorhergehenden Ansprüche, **dadurch gekennzeichnet, dass** der Oxidationsfarbstoff unter den Oxidationsbasen und/oder Kupplern ausgewählt ist.

14. Zusammensetzung nach Anspruch 13, **dadurch gekennzeichnet, dass** sie mindestens eine Oxidationsbase enthält.

15. Zusammensetzung nach Anspruch 14, **dadurch gekennzeichnet, dass** die Oxidationsbasen unter den o- oder p-Phenylendiaminen, Doppelbasen, o- oder p-Aminophenolen und den heterocyclischen Basen sowie den Additionssalzen dieser Verbindungen mit einer Säure ausgewählt sind.

16. Zusammensetzung nach Anspruch 15, **dadurch gekennzeichnet, dass** die p-Phenylendiamine unter den Verbindungen der folgenden Struktur (III) ausgewählt sind:

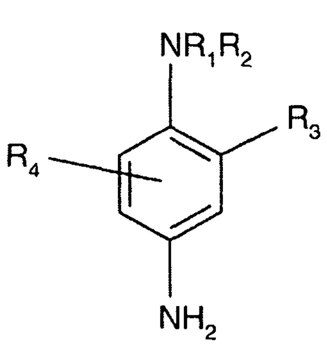

**(III)**

worin bedeuten:

$R_1$ ein Wasserstoffatom, Alkyl($C_{1-4}$), Monohydroxyalkyl($C_{1-4}$), Polyhydroxyalkyl($C_{2-4}$), Alkoxy($C_{1-4}$)alkyl($C_{1-4}$),

eine mit einer stickstoffhaltigen Gruppe substituierte $C_{1-4}$-Alkylgruppe, Phenyl oder 4'-Aminophenyl;
$R_2$ ein Wasserstoffatom, Alkyl($C_{1-4}$), Monohydroxyalkyl($C_{1-4}$), Polyhydroxyalkyl($C_{2-4}$), Alkoxy($C_{1-4}$)alkyl($C_{1-4}$) oder mit einer stickstoffhaltigen Gruppe substituierte $C_{1-4}$-Alkylgruppe;
$R_1$ und $R_2$ können auch mit dem Stickstoffatom, das sie trägt, einen 5- oder 6-gliedrigen Stickstoffheterocyclus bilden, der gegebenenfalls mit einer oder mehreren Gruppen Alkyl, Hydroxy oder Ureido substituiert ist;
$R_3$ ein Wasserstoffatom, ein Halogenatom, wie ein Chloratom, Alkyl($C_{1-4}$), Sulfo, Carboxy, Monohydroxyalkyl ($C_{1-4}$), Hydroxyalkoxy($C_{1-4}$), Acetylaminoalkoxy($C_{1-4}$), Mesylaminoalkoxy($C_{1-4}$) oder Carbamoylaminoalkoxy ($C_{1-4}$);
$R_4$ ein Wasserstoffatom, ein Halogenatom oder Alkyl($C_{1-4}$).

**17.** Zusammensetzung nach Anspruch 15, **dadurch gekennzeichnet, dass** die Doppelbasen unter den Verbindungen der folgenden Struktur (IV) ausgewählt sind:

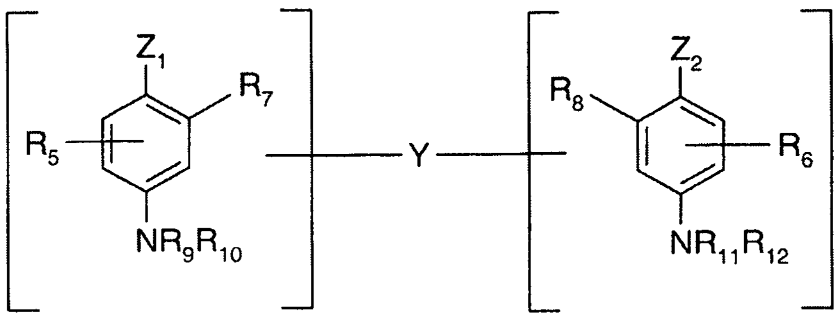

(IV)

worin bedeuten:

- $Z_1$ und $Z_2$, die gleich oder verschieden sind, eine Gruppe Hydroxy oder $-NH_2$, die mit einer $C_{1-4}$-Alkylgruppe oder einer Verbindungsgruppe Y substituiert sein kann;
- die Verbindungsgruppe Y eine geradkettige oder verzweigte Alkylenkette mit 1 bis 14 Kohlenstoffatomen, die durch eine oder mehrere stickstoffhaltige Gruppen und/oder durch ein oder mehrere Heteroatome, wie Sauerstoff, Schwefel oder Stickstoff, unterbrochen oder abgeschlossen werden kann und gegebenenfalls mit einer oder mehreren Hydroxygruppen oder $C_{1-6}$-Alkoxygruppen substituiert ist;
- $R_5$ und $R_6$ ein Wasserstoffatom, ein Halogenatom, Alkyl($C_{1-4}$), Monohydroxyalkyl($C_{1-4}$), Polyhydroxyalkyl ($C_{2-4}$), Aminoalkyl($C_{1-4}$) oder eine Verbindungsgruppe Y;
- $R_7$, $R_8$, $R_9$, $R_{10}$, $R_{11}$ und $R_{12}$, die gleich oder verschieden sind, ein Wasserstoffatom, eine Verbindungsgruppe Y oder $C_{1-4}$-Alkyl;

mit der Maßgabe, dass die Verbindungen der Formel (IV) nur eine Verbindungsgruppe Y pro Molekül aufweisen.

**18.** Zusammensetzung nach Anspruch 15, **dadurch gekennzeichnet, dass** die p-Aminophenole unter den Verbindungen der folgenden Struktur (V) ausgewählt sind:

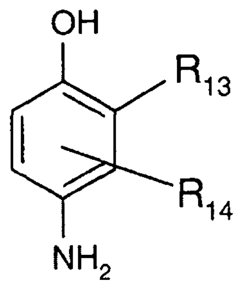

(V)

worin bedeuten:

$R_{13}$ ein Wasserstoffatom, ein Halogenatom, wie Fluor, Alkyl($C_{1-4}$), Monohydroxyalkyl($C_{1-4}$), Alkoxy($C_{1-4}$)alkyl ($C_{1-4}$), Aminoalkyl($C_{1-4}$) oder Hydroxyalkyl($C_{1-4}$)aminoalkyl($C_{1-4}$),
$R_{14}$ ein Wasserstoffatom, ein Halogenatom, wie Fluor, Alkyl($C_{1-4}$), Monohydroxyalkyl($C_{1-4}$), Polyhydroxyalkyl ($C_{2-4}$), Aminoalkyl($C_{1-4}$), Cyanoalkyl($C_{1-4}$) oder Alkoxy($C_{1-4}$)alkyl($C_{1-4}$).

**19.** Zusammensetzung nach Anspruch 15, **dadurch gekennzeichnet, dass** die heterocyclischen Basen unter den Pyridinderivaten, Pyrimidinderivaten und darunter Pyrazolopyrimidinen und Pyrazolderivaten ausgewählt sind.

**20.** Zusammensetzung nach einem der Ansprüche 13 bis 19, **dadurch gekennzeichnet, dass** die Oxidationsbasen in Konzentrationen von 0,0005 bis 12 Gew.-%, bezogen auf das Gesamtgewicht der Zusammensetzung, vorliegen.

**21.** Zusammensetzung nach Anspruch 13, **dadurch gekennzeichnet, dass** die Kuppler unter *m*-Phenylendiaminen, *m*-Aminophenolen, *m*-Dihydroxybenzolen, heterocyclischen Kupplern und den Additionssalzen dieser Verbindungen mit einer Säure ausgewählt sind.

**22.** Zusammensetzung nach einem der Ansprüche 13 oder 21, **dadurch gekennzeichnet, dass** die Kuppler in Konzentrationen von 0,0001 bis 10 Gew.-%, bezogen auf das Gesamtgewicht der Zusammensetzung, enthalten sind.

**23.** Zusammensetzung nach einem der Ansprüche 13 bis 22, **dadurch gekennzeichnet, dass** die Additionssalze der Oxidationsfarbstoffe mit einer Säure unter den Hydrochloriden, Hydrobromiden, Sulfaten, Tartraten, Lactaten und Acetaten ausgewählt sind.

**24.** Zusammensetzung nach einem der vorhergehenden Ansprüche, **dadurch gekennzeichnet, dass** der Direktfarbstoff unter den neutralen, sauren oder kationischen, nitrierten direktziehenden Benzolfarbstoffen, neutralen, sauren oder kationischen direktziehenden Azofarbstoffen, direktziehenden Chinonfarbstoffen, direktziehenden Azin-Farbstoffen, direktziehenden Triarylmethan-Farbstoffen, direktziehenden Indoamin-Farbstoffen und den direktziehenden natürlichen Farbstoffen ausgewählt ist.

**25.** Zusammensetzung nach dem vorhergehenden Anspruch, **dadurch gekennzeichnet, dass** der Direktfarbstoff unter den neutralen, sauren oder kationischen direktziehenden Anthrachinon-Farbstoffen ausgewählt ist.

**26.** Zusammensetzung nach einem der vorhergehenden Ansprüche, **dadurch gekennzeichnet, dass** der oder die Direktfarbstoff in Konzentrationen im Bereich von 0,001 bis 20 Gew.-%, bezogen auf das Gesamtgewicht der Zusammensetzung, enthalten sind.

**27.** Zusammensetzung nach einem der vorhergehenden Ansprüche, **dadurch gekennzeichnet, dass** sie ferner mindestens ein Reduktionsmittel in Mengenanteilen von 0,05 bis 3 Gew.-%, bezogen auf das Gesamtgewicht der Zusammensetzung, enthält.

**28.** Zusammensetzung nach einem der vorhergehenden Ansprüche, **dadurch gekennzeichnet, dass** sie ferner ein Oxidationsmittel enthält und **dadurch**, dass sie gebrauchsfertig ist.

**29.** Zusammensetzung nach Anspruch 28, **dadurch gekennzeichnet, dass** das Oxidationsmittel unter Wasserstoffperoxid, Harnstoffperoxid, Alkalimetallbromaten, Alkalimetallferricyaniden, Salzen von Persäuren, Redoxenzymen gegebenenfalls zusammen mit ihrem jeweiligen Donor oder Cofaktor ausgewählt ist.

**30.** Zusammensetzung nach Anspruch 29, **dadurch gekennzeichnet, dass** es sich bei dem Oxidationsmittel um Wasserstoffperoxid handelt.

**31.** Zusammensetzung nach Anspruch 30, **dadurch gekennzeichnet, dass** das Oxidationsmittel eine Wasserstoffperoxidlösung ist, deren Titer im Bereich von 1 bis 40 Volumina liegt.

**32.** Zusammensetzung nach einem der vorhergehenden Ansprüche, **dadurch gekennzeichnet, dass** sie einen pH-Wert von 4 bis 11 aufweist.

**33.** Verfahren zum Färben menschlicher Keratinfasern, **dadurch gekennzeichnet, dass** es darin besteht, auf die Fasern eine Farbmittelzusammensetzung aufzutragen, die in einem kosmetisch akzeptablen Medium mindestens einen Direktfarbstoff und ferner mindestens ein aminiertes Silicon der Formel (I) oder (II), wie es in einem der

Ansprüche 1 bis 12 definiert ist, enthält.

**34.** Verfahren zum Färben menschlicher Keratinfasern, **dadurch gekennzeichnet, dass** es darin besteht, auf die Fasern mindestens eine Farbmittelzusammensetzung aufzutragen, die in einem kosmetisch akzeptablen Medium mindestens einen Direktfarbstoff oder mindestens einen Oxidationsfarbstoff und ferner mindestens ein aminiertes Silicon der Formel (I) oder (II), wie es in einem der Ansprüche 1 bis 12 definiert ist, enthält, wobei die Farbe bei einem alkalischen, neutralen oder sauren pH-Wert mit einer Zusammensetzung gebildet wird, die mindestens ein Oxidationsmittel enthält und die bei der Anwendung mit der Farbmittelzusammensetzung vermischt wird oder die ohne zwischenzeitliches Spülen anschließend aufgetragen wird.

**35.** Verfahren nach Anspruch 34, **dadurch gekennzeichnet, dass** es darin besteht, auf die trockenen oder feuchten Keratinfasern die Farbmittelzusammensetzung oder die gebrauchsfertige Zusammensetzung aufzutragen, die bedarfsgemäß bei der Anwendung aus der Farbmittelzusammensetzung und der oxidierenden Zusammensetzung gebildet wird, diese während einer Einwirkzeit von etwa 1 bis 60 Minuten einwirken zu lassen, die Fasern zu spülen, gegebenenfalls mit Haarwaschmittel zu waschen und nochmals zu spülen und sie zu trocknen.

**36.** Vorrichtung mit zwei Abteilungen oder «Kit» zum Färben von menschlichen Keratinfasern, **dadurch gekennzeichnet, dass** sie eine Abteilung aufweist, die eine Zusammensetzung enthält, die in einem kosmetisch akzeptablen Medium mindestens einen Direktfarbstoff oder mindestens einen Oxidationsfarbstoff und mindestens ein aminiertes Silicon der Formel (I) oder (II), wie es in einem der Ansprüche 1 bis 12 definiert ist, enthält, und eine andere Abteilung aufweist, die eine Zusammensetzung enthält, die in einem zum Färben geeigneten Medium ein Oxidationsmittel enthält.

**37.** Vorrichtung mit drei Abteilung oder «Kit» zum Färben von menschlichen Keratinfasern, **dadurch gekennzeichnet, dass** sie eine erste Abteilung aufweist, die eine Zusammensetzung enthält, die in einem kosmetisch akzeptablen Medium mindestens einen Direktfarbstoff oder mindestens einen Oxidationsfarbstoff aufweist, eine zweite Abteilung, die eine Zusammensetzung enthält, die in einem kosmetisch akzeptablen Medium mindestens ein Oxidationsmittel enthält, und eine dritte Abteilung, die eine Zusammensetzung enthält, die in einem kosmetisch akzeptablen Medium mindestens ein aminiertes Silicon der Formel (I) oder (II), wie es in einem der Ansprüche 1 bis 12 definiert ist, enthält.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- EP 11138317 A
- GB 1026978 A
- GB 1153196 A
- DE 2359399
- JP 63169571 A
- JP 3010659 A
- WO 9615765 A
- FR 2750048 A
- DE 3843892
- DE 4133957
- WO 9408969 A
- WO 9408970 A
- FR 2733749 A
- DE 19543988
- WO 9515144 A
- WO 9501772 A
- EP 714954 A
- US 2528378 A
- US 2781354 A
- EP 337354 A
- FR 2270846
- FR 2383660
- FR 2598611
- FR 2470596
- FR 2519863
- FR 2505348
- FR 2542997
- EP 080976 A
- FR 2077143
- FR 2393573
- FR 1492597
- US 4131576 A
- US 3589578 A
- US 4031307 A
- FR 2162025
- FR 2280361
- FR 2252840
- FR 2368508
- FR 1583363
- US 3227615 A
- US 2961347 A
- FR 2080759
- FR 2190406
- FR 23203302270846
- FR 2316271
- FR 2336434
- FR 2413907
- US 2273780 A
- US 2375853 A
- US 2388614 A
- US 2454547 A
- US 3206462 A
- US 2261002 A
- US 2271378 A
- US 3874870 A
- US 4001432 A
- US 3929990 A
- US 3966904 A
- US 4005193 A
- US 4025617 A
- US 4025627 A
- US 4025653 A
- US 4026945 A
- US 4027020 A
- US 4157388 A
- US 4702906 A
- US 4719282 A
- EP 122324 A
- FR 1400366


### Littérature non-brevet citée dans la description


- **M.R. PORTER.** Handbook of Surfactants. Blackie & Son, 1991, 116-178
- CTFA. 1993